# EUROPEAN PATENT SPECIFICATION

(11) **EP 3 417 061 B1**
(45) Date of publication and mention of the grant of the patent: **26.10.2022**
(21) Application number: 17753841.0
(22) Date of filing: 16.02.2017
(51) Int. Cl.: C12N 9/22, C12N 15/10, C07K 14/47

(54) **METHODS AND COMPOSITIONS FOR GENE EDITING IN STEM CELLS**
VERFAHREN UND ZUSAMMENSETZUNGEN ZUR GENEDITIERUNG BEI STAMMZELLEN
MÉTHODES ET COMPOSITIONS POUR L'ÉDITION DE GÈNES DANS DES CELLULES SOUCHES

(30) Priority: 18.02.2016 US 201662297039 P
(43) Date of publication of application: 26.12.2018
(73) Proprietor: The Regents of the University of California, Oakland, CA 94607-5200 (US)
(72) Inventor: KOHN, Donald B., East Los Angeles, CA 90095 (US); BJURSTROEM, Carmen Flores, East Los Angeles, CA 90095 (US)
(74) Representative: Mewburn Ellis LLP
(86) International application number: PCT/US2017/018183
(87) International publication number: WO 2017/143071

(56) References cited:
- US-A1- 2015 132 269
- US-A1- 2015 232 833
- US-A1- 2016 024 474
- CHAO D T ET AL: "BCL-2 FAMILY: REGULATORS OF CELL DEATH", ANNUAL REVIEW OF IMMUNOLOGY, ANNUAL REVIEWS INC, vol. 16, 1 January 1998 (1998-01-01), pages 395-419, XP001093719, ISSN: 0732-0582, DOI: 10.1146/ANNUREV.IMMUNOL.16.1.395
- XIAO-LAN LI ET AL: "Highly efficient genome editing via CRISPR-Cas9 in human pluripotent stem cells is achieved by transient BCL-XL overexpression", NUCLEIC ACIDS RESEARCH, vol. 46, no. 19, 20 September 2018 (2018-09-20), pages 10195-10215, XP055605965, ISSN: 0305-1048, DOI: 10.1093/nar/gky804
- CARMEN F BJURSTROEM ET AL: "3012 -IDENTIFYING MECHANISMS THAT LIMIT EFFICIENT SITE- SPECIFIC GENE MODIFICATION BY HOMOLOGY-DIRECTED REPAIR IN HEMATOPOIETIC STEM CELLS", EXPERIMENTAL HEMATOLOGY, 1 January 2015 (2015-01-01), page S54, XP055605729,
- HOBAN MEGAN D ET AL: "Evaluation of TALENs and CRISPR/Cas9 nuclease system to correct the sickle cell disease mutation", MOLECULAR THERAPY, 18TH ANNUAL MEETING OF THE AMERICAN-SOCIETY-OF-GENE-AND-CELL-THERAPY (ASGCT); NEW ORLEANS, LA, USA; MAY 13 -16, 2015, vol. 23, no. Suppl. 1, 1 May 2015 (2015-05-01), page S135, XP002761325,
- KIHEON BAEK ET AL: "Gene Transfection for Stem Cell Therapy", CURRENT STEM CELL REPORTS, vol. 2, no. 1, 27 January 2016 (2016-01-27), pages 52-61, XP055605977, DOI: 10.1007/s40778-016-0029-5
- SPEAKERS: "The European Society for Gene and Cell Therapy and the Spanish Society for Gene and Cell Therapy Collaborative Congress 2013 Conference Abstracts Palacio Municipal de Congresos Madrid, Spain October 25-28, 2013", HUMAN GENE THERAPY, vol. 24, no. 12, 1 December 2013 (2013-12-01), pages A1-A172, XP055463206, US ISSN: 1043-0342, DOI: 10.1089/hum.2013.2513
- CHIA-WEI CHANG ET AL: "Modeling Human Severe Combined Immunodeficiency and Correction by CRISPR/Cas9-Enhanced Gene Targeting", CELL REPORTS, vol. 12, no. 10, 1 September 2015 (2015-09-01), pages 1668-1677, XP055606304, US ISSN: 2211-1247, DOI: 10.1016/j.celrep.2015.08.013
- FANG ZHONG ET AL: "Differentiation of GFP-Bcl-2-engineered mesenchymal stem cells towards a nucleus pulposus-like phenotype under hypoxiain vitro", BIOCHEMICAL AND BIOPHYSICAL RESEARCH COMMUNICATIONS, ELSEVIER, AMSTERDAM, NL, vol. 432, no. 3, 12 February 2013 (2013-02-12), pages 444-450, XP028998193, ISSN: 0006-291X, DOI: 10.1016/J.BBRC.2013.01.127
- Carmen Flores Bjurström ET AL: "Preservation of Gene Edited Hematopoietic Stem Cells By Transient Overexpression of BCL-2 mRNA", Blood, 2 December 2016 (2016-12-02), page 3636, XP055606756, Retrieved from the Internet: URL:http://www.bloodjournal.org/content/12 8/22/3636 [retrieved on 2019-07-18]
- GENOVESE ET AL.: 'Targeted genome editing in human repopulating haematopoietic stem cells' NATURE vol. 510, no. 7504, pages 235 - 240, XP055277712
- HENDRIKS ET AL.: 'Genome editing in human pluripotent stem cells:approaches, pitfalls, and solutions' CELL STEM CELL vol. 18, no. 1, 07 January 2016, pages 53 - 65, XP029381742
- VASILEVA ET AL.: 'Genome-editing tools for stem cell biology' CELL DEATH AND DISEASE vol. 6, 23 July 2015, pages 1 - 8, XP055410038

## Description

### BACKGROUND

RNA-mediated adaptive immune systems in bacteria and archaea rely on Clustered Regularly Interspaced Short Palindromic Repeat (CRISPR) genomic loci and CRISPR-associated (Cas) proteins that function together to provide protection from invading viruses and plasmids. In Type II CRISPR-Cas systems, the Cas9 protein functions as an RNA-guided endonuclease that uses a dual-guide RNA consisting of crRNA and *trans-*activating crRNA (tracrRNA) for target recognition and cleavage by a mechanism involving two nuclease active sites that together generate double-stranded DNA breaks (DSBs).

RNA-programmed Cas9 has proven to be a versatile tool for genome engineering in multiple cell types and organisms. Guided by a dual-RNA complex or a chimeric single-guide RNA, Cas9 (or variants of Cas9 such as nickase variants) can generate site-specific DSBs or single-stranded breaks (SSBs) within target nucleic acids. Target nucleic acids can include double-stranded DNA (dsDNA) and single-stranded DNA (ssDNA) as well as RNA. When cleavage of a target nucleic acid occurs within a cell *(e.g.,* a eukaryotic cell), the break in the target nucleic acid can be repaired by non-homologous end joining (NHEJ) or HDR.

Disruption of mammalian genes holds great promise for fundamental discovery, treatment of genetic diseases, and prophylactic treatment. Gene knockouts can be generated using a genome editing endonuclease *(e.g.,* a zinc finger nuclease (ZFN), a transcription activator-like effector nuclease (TALEN), a CRISPR/Cas protein:guide RNA, and the like) to introduce a site specific double strand break (DSB) within a locus *(e.g.,* gene) of interest. Clones can be screened for those in which one or more alleles have been repaired in an error-prone fashion that disrupts the open reading frame.

Gene correction can be achieved in stem cells (*e.g.,* CD34⁺ stem cells) and progenitor cells; nevertheless, levels of homology-directed repair (HDR)-mediated gene modification in long-term reconstituting stem cells, such as hematopoietic stem cells (HSCs), remain low.

Bjurström et al. (2015) Experimental Hematology 43(9): S54; Hoban et al. (2015) Molecular Therapy 23(Supplement 1): S135; Baek et al. (2016) Current Stem Cell Reports 2(1):52-61; Speakers (2013) Human Gene Therapy 24(12):A1-A172; Chang et al. (2015) Cell Reports 12(1): 1668-1677; US 2015/232833; US 2016/024474; Vasileva et al. (2015) Cell Death and Disease 6: 1-8; Hendriks et al. (2016) Cell Stem Cell 18(1): 53-65; and Genovese et al. (2014) Nature 510(7504): 235-240 describe methods of *ex vivo* editing of a target genomic step cell comprising contacting the mammalian stem cell with a genome targeting composition.

### SUMMARY

The present disclosure provides, *inter alia,* compositions and methods for gene editing in stem cells. The methods generally involve modifying the stem cells by increasing the level of an apoptosis regulator in the stem cells; and introducing into the modified stem cells a genome editing composition.

The invention is set out in the claims.

### BRIEF DESCRIPTION OF THE DRAWINGS

Figures 1A and 1B provide amino acid sequences of Bcl-2 alpha (Fig. 1A) (SEQ ID NO:1142) and Bcl-2 beta (Fig. 1B) (SEQ ID NO:1143).
Figure 2 provides an amino acid sequence of Mcl-1 (SEQ ID NO:1144).
Figure 3 provides an amino acid sequence of survivin (SEQ ID NO:1145).

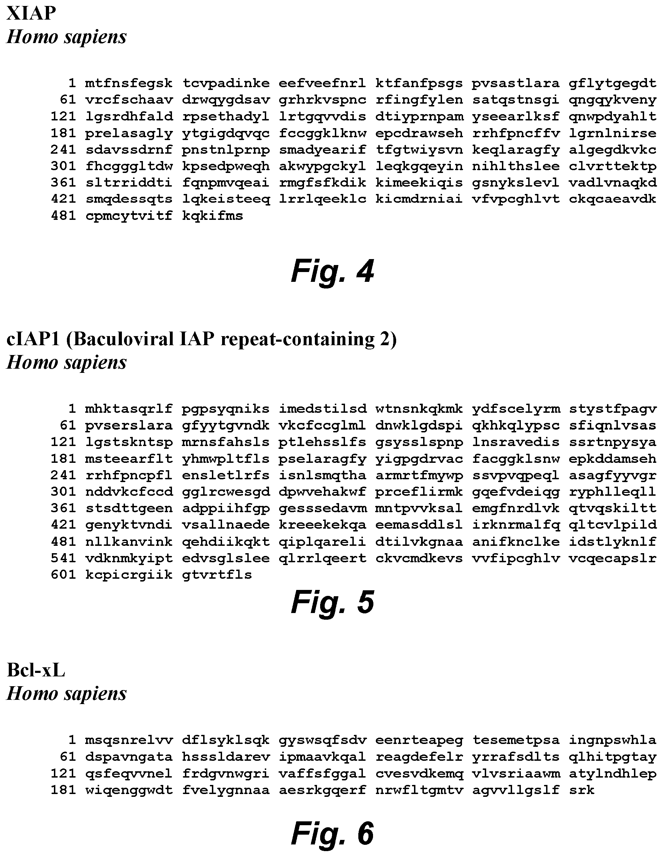

Figure 4 provides an amino acid sequence of XIAP (SEQ ID NO:1146).
Figure 5 provides an amino acid sequence of cIAP1 (SEQ ID NO:1147).
Figure 6 provides an amino acid sequence of Bcl-xL (SEQ ID NO:1148).
Figure 7 provides a schematic depiction of the experimental design, and immunophenotype-defined populations.
Figure 8 provides a HDR vs. NHEJ comparison in various cell populations.
Figure 9 depicts cell death and apoptosis analysis of various cell populations post-electroporation.
Figures 10A-10C depict the effect of transient overexpression of BCL-2 mRNA on cell toxicity as measured by flow cytometry and on the number of cells overall.
Figure 11 depicts the effect of transient overexpression of BCL-2 mRNA on gene modification levels in HSC, MPP, and progenitor cells treated with ZFN mRNA and an oligonucleotide donor.
Figures 12A-12B depict apoptosis pathway gene expression analysis by qPCR in human HSCs, MPPs and progenitor cells.
Figures 13A-13D depict xenograft transplantations of female immunedeficient NSG mice.

### Definitions

By "site-directed modifying polypeptide" or "site-directed DNA modifying polypeptide" or "site-directed target nucleic acid modifying polypeptide" or "RNA-binding site-directed polypeptide" or "RNA-binding site-directed modifying polypeptide" or "site-directed polypeptide" it is meant a polypeptide that binds a guide RNA and is targeted to a specific DNA sequence by the guide RNA. A site-directed modifying polypeptide can be class 2 CRISPR/Cas protein *(e.g.,* a type II CRISPR/Cas protein, a type V CRISPR/Cas protein, a type VI CRISPR/Cas protein). An example of a type II CRISPR/Cas protein is a Cas9 protein ("Cas9 polypeptide"). Examples of type V CRISPR/Cas proteins are Cpfl, C2c1, and C2c3. An example of a type II CRISPR/Cas protein is a C2c2 protein. Class 2 CRISPR/Cas proteins (*e.g.*, Cas9, Cpfl, C2c1, C2c2, and C2c3) as described herein are targeted to a specific DNA sequence by the RNA (a guide RNA) to which it is bound. The guide RNA comprises a sequence that is complementary to a target sequence within the target DNA, thus targeting the bound CRISPR/Cas protein to a specific location within the target DNA (the target sequence). For example, a Cpfl polypeptide as described herein is targeted to a specific DNA sequence by the RNA (a guide RNA) to which it is bound. The guide RNA comprises a sequence that is complementary to a target sequence within the target DNA, thus targeting the bound Cpfl protein to a specific location within the target DNA (the target sequence).

"Heterologous," as used herein, means a nucleotide or polypeptide sequence that is not found in the native nucleic acid or protein, respectively.

The terms "polynucleotide" and "nucleic acid," used interchangeably herein, refer to a polymeric form of nucleotides of any length, either ribonucleotides or deoxynucleotides. Thus, this term includes, but is not limited to, single-, double-, or multi-stranded DNA or RNA, genomic DNA, cDNA, DNA-RNA hybrids, or a polymer comprising purine and pyrimidine bases or other natural, chemically or biochemically modified, non-natural, or derivatized nucleotide bases. The terms "polynucleotide" and "nucleic acid" should be understood to include, as applicable to the embodiment being described, single-stranded (such as sense or antisense) and double-stranded polynucleotides.

The term "naturally-occurring" as used herein as applied to a nucleic acid, a cell, or an organism, refers to a nucleic acid, cell, or organism that is found in nature. For example, a polypeptide or polynucleotide sequence that is present in an organism (including viruses) that can be isolated from a source in nature and which has not been intentionally modified by a human in the laboratory is naturally occurring.

As used herein the term "isolated" is meant to describe a polynucleotide, a polypeptide, or a cell that is in an environment different from that in which the polynucleotide, the polypeptide, or the cell naturally occurs. An isolated genetically modified host cell may be present in a mixed population of genetically modified host cells.

As used herein, the term "exogenous nucleic acid" refers to a nucleic acid that is not normally or naturally found in and/or produced by a given cell in nature. As used herein, the term "endogenous nucleic acid" refers to a nucleic acid that is normally found in and/or produced by a given cell in nature. An "endogenous nucleic acid" is also referred to as a "native nucleic acid" or a nucleic acid that is "native" to a given cell.

"Recombinant," as used herein, means that a particular nucleic acid (DNA or RNA) is the product of various combinations of cloning, restriction, and/or ligation steps resulting in a construct having a structural coding or non-coding sequence distinguishable from endogenous nucleic acids found in natural systems. Generally, DNA sequences encoding the structural coding sequence can be assembled from cDNA fragments and short oligonucleotide linkers, or from a series of synthetic oligonucleotides, to provide a synthetic nucleic acid which is capable of being expressed from a recombinant transcriptional unit contained in a cell or in a cell-free transcription and translation system. Such sequences can be provided in the form of an open reading frame uninterrupted by internal non-translated sequences, or introns, which are typically present in eukaryotic genes. Genomic DNA comprising the relevant sequences can also be used in the formation of a recombinant gene or transcriptional unit. Sequences of non-translated DNA may be present 5' or 3' from the open reading frame, where such sequences do not interfere with manipulation or expression of the coding regions, and may indeed act to modulate production of a desired product by various mechanisms (see "DNA regulatory sequences", below).

Thus, *e.g.,* the term "recombinant" polynucleotide or "recombinant" nucleic acid refers to one which is not naturally occurring, *e.g.,* is made by the artificial combination of two otherwise separated segments of sequence through human intervention. This artificial combination is often accomplished by either chemical synthesis means, or by the artificial manipulation of isolated segments of nucleic acids, *e.g.,* by genetic engineering techniques. Such can be done to replace a codon with a redundant codon encoding the same or a conservative amino acid, while typically introducing or removing a sequence recognition site. It can also be performed to join together nucleic acid segments of desired functions to generate a desired combination of functions. This artificial combination is often accomplished by either chemical synthesis means, or by the artificial manipulation of isolated segments of nucleic acids, *e.g.,* by genetic engineering techniques.

Similarly, the term "recombinant" polypeptide refers to a polypeptide which is not naturally occurring, *e.g.,* is made by the artificial combination of two otherwise separated segments of amino sequence through human intervention. Thus, *e.g.,* a polypeptide that comprises a heterologous amino acid sequence is recombinant.

By "recombination" it is meant a process of exchange of genetic information between two polynucleotides. As used herein, "homology-directed repair (HDR)" refers to the specialized form DNA repair that takes place, for example, during repair of double-strand breaks in cells. This process requires nucleotide sequence homology, uses a "donor" molecule to template repair of a "target" molecule (*i.e.,* the one that experienced the double-strand break), and leads to the transfer of genetic information from the donor to the target. Homology-directed repair may result in an alteration of the sequence of the target molecule (*e.g*., insertion, deletion, mutation), if the donor polynucleotide differs from the target molecule and part or all of the sequence of the donor polynucleotide is incorporated into the target DNA. In some embodiments, the donor polynucleotide, a portion of the donor polynucleotide, a copy of the donor polynucleotide, or a portion of a copy of the donor polynucleotide integrates into the target DNA.

By "non-homologous end joining (NHEJ)" it is meant the repair of double-strand breaks in DNA by direct ligation of the break ends to one another without the need for a homologous template (in contrast to homology-directed repair, which requires a homologous sequence to guide repair). NHEJ often results in the loss (deletion) of nucleotide sequence near the site of the double-strand break.

By "construct" or "vector" is meant a recombinant nucleic acid, generally recombinant DNA, which has been generated for the purpose of the expression and/or propagation of a specific nucleotide sequence(s), or is to be used in the construction of other recombinant nucleotide sequences.

The terms "DNA regulatory sequences," "control elements," and "regulatory elements," used interchangeably herein, refer to transcriptional and translational control sequences, such as promoters, enhancers, polyadenylation signals, terminators, protein degradation signals, and the like, that provide for and/or regulate expression of a coding sequence and/or production of an encoded polypeptide in a host cell.

The term "transformation" is used interchangeably herein with "genetic modification" and refers to a permanent or transient genetic change induced in a cell following introduction of new nucleic acid *(i.e.,* DNA exogenous to the cell). Genetic change ("modification") can be accomplished either by incorporation of the new DNA into the genome of the host cell, or by transient or stable maintenance of the new DNA as an episomal element. Where the cell is a eukaryotic cell, a permanent genetic change is generally achieved by introduction of the DNA into the genome of the cell.

"Operably linked" refers to a juxtaposition wherein the components so described are in a relationship permitting them to function in their intended manner. For instance, a promoter is operably linked to a coding sequence if the promoter affects its transcription or expression. As used herein, the terms "heterologous promoter" and "heterologous control regions" refer to promoters and other control regions that are not normally associated with a particular nucleic acid in nature. For example, a "transcriptional control region heterologous to a coding region" is a transcriptional control region that is not normally associated with the coding region in nature.

A "host cell," as used herein, denotes an *in vivo* or *in vitro* eukaryotic cell, or a cell from a multicellular organism (*e.g*., a cell line) cultured as a unicellular entity, which eukaryotic cells can be, or have been, used as recipients for a nucleic acid *(e.g.,* a donor template nucleic acid), and include the progeny of the original cell which has been genetically modified by the nucleic acid. It is understood that the progeny of a single cell may not necessarily be completely identical in morphology or in genomic or total DNA complement as the original parent, due to natural, accidental, or deliberate mutation. A "recombinant host cell" (also referred to as a "genetically modified host cell") is a host cell into which has been introduced a heterologous nucleic acid, *e.g.,* an expression vector. For example, a eukaryotic host cell is a genetically modified eukaryotic host cell, by virtue of introduction into a suitable eukaryotic host cell of a heterologous nucleic acid, *e.g.,* an exogenous nucleic acid that is foreign to the eukaryotic host cell, or a recombinant nucleic acid that is not normally found in the eukaryotic host cell.

The term "stem cell" is used herein to refer to a cell *(e.g.,* plant stem cell, vertebrate stem cell) that has the ability both to self-renew and to generate a differentiated cell type (see Morrison et al. (1997) Cell 88:287-298). In the context of cell ontogeny, the adjective "differentiated", or "differentiating" is a relative term. A "differentiated cell" is a cell that has progressed further down the developmental pathway than the cell it is being compared with. Thus, pluripotent stem cells (described below) can differentiate into lineage-restricted progenitor cells (*e.g*., mesodermal stem cells), which in turn can differentiate into cells that are further restricted (*e.g*., neuron progenitors), which can differentiate into end-stage cells (*i.e.,* terminally differentiated cells, *e.g.,* neurons, cardiomyocytes, *etc.),* which play a characteristic role in a certain tissue type, and may or may not retain the capacity to proliferate further. Stem cells may be characterized by both the presence of specific markers *(e.g.,* proteins, RNAs, *etc.)* and the absence of specific markers. Stem cells may also be identified by functional assays both *in vitro* and *in vivo,* particularly assays relating to the ability of stem cells to give rise to multiple differentiated progeny.

Stem cells of interest include pluripotent stem cells (PSCs). The term "pluripotent stem cell" or "PSC" is used herein to mean a stem cell capable of producing all cell types of the organism. Therefore, a PSC can give rise to cells of all germ layers of the organism (*e.g*., the endoderm, mesoderm, and ectoderm of a vertebrate). Pluripotent cells are capable of forming teratomas and of contributing to ectoderm, mesoderm, or endoderm tissues in a living organism. Pluripotent stem cells of plants are capable of giving rise to all cell types of the plant *(e.g.,* cells of the root, stem, leaves, *etc.).*

PSCs of animals can be derived in a number of different ways. For example, induced pluripotent stem cells (iPSCs) are derived from somatic cells (Takahashi et. al, Cell. 2007 Nov 30;131(5):861-72; Takahashi et. al, Nat Protoc. 2007;2(12):3081-9; Yu et. al, Science. 2007 Dec 21;318(5858):1917-20. Epub 2007 Nov 20).

By "induced pluripotent stem cell" or "iPSC" it is meant a PSC that is derived from a cell that is not a PSC (*i.e.,* from a cell this is differentiated relative to a PSC). iPSCs can be derived from multiple different cell types, including terminally differentiated cells. iPSCs have an ES cell-like morphology, growing as flat colonies with large nucleo-cytoplasmic ratios, defined borders and prominent nuclei. In addition, iPSCs express one or more key pluripotency markers known by one of ordinary skill in the art, including but not limited to Alkaline Phosphatase, SSEA3, SSEA4, Sox2, Oct3/4, Nanog, TRA160, TRA181, TDGF 1, Dnmt3b, FoxD3, GDF3, Cyp26a1, TERT, and zfp42. Examples of methods of generating and characterizing iPSCs may be found in, for example, U.S. Patent Publication Nos. US20090047263, US20090068742, US20090191159, US20090227032, US20090246875, and US20090304646. Generally, to generate iPSCs, somatic cells are provided with reprogramming factors *(e.g.,* Oct4, SOX2, KLF4, MYC, Nanog, Lin28, *etc.)* known in the art to reprogram the somatic cells to become pluripotent stem cells.

By "somatic cell" it is meant any cell in an organism that, in the absence of experimental manipulation, does not ordinarily give rise to all types of cells in an organism. In other words, somatic cells are cells that have differentiated sufficiently that they will not naturally generate cells of all three germ layers of the body, *i.e.* ectoderm, mesoderm and endoderm. For example, somatic cells would include both neurons and neural progenitors, the latter of which may be able to naturally give rise to all or some cell types of the central nervous system but cannot give rise to cells of the mesoderm or endoderm lineages.

The term "conservative amino acid substitution" refers to the interchangeability in proteins of amino acid residues having similar side chains. For example, a group of amino acids having aliphatic side chains consists of glycine, alanine, valine, leucine, and isoleucine; a group of amino acids having aliphatic-hydroxyl side chains consists of serine and threonine; a group of amino acids having amide-containing side chains consists of asparagine and glutamine; a group of amino acids having aromatic side chains consists of phenylalanine, tyrosine, and tryptophan; a group of amino acids having basic side chains consists of lysine, arginine, and histidine; and a group of amino acids having sulfur-containing side chains consists of cysteine and methionine. Exemplary conservative amino acid substitution groups are: valine-leucine-isoleucine, phenylalanine-tyrosine, lysine-arginine, alanine-valine, and asparagine-glutamine.

A polynucleotide or polypeptide has a certain percent "sequence identity" to another polynucleotide or polypeptide, meaning that, when aligned, that percentage of bases or amino acids are the same, and in the same relative position, when comparing the two sequences. Sequence similarity can be determined in a number of different manners. To determine sequence identity, sequences can be aligned using the methods and computer programs, including BLAST, available over the world wide web at ncbi.nlm.nih.gov/BLAST. See, *e.g.,* Altschul et al. (1990), J. Mol. Biol. 215:403-10. Another alignment algorithm is FASTA, available in the Genetics Computing Group (GCG) package, from Madison, Wisconsin, USA, a wholly owned subsidiary of Oxford Molecular Group, Inc. Other techniques for alignment are described in Methods in Enzymology, vol. 266: Computer Methods for Macromolecular Sequence Analysis (1996), ed. Doolittle, Academic Press, Inc., a division of Harcourt Brace & Co., San Diego, California, USA. Of particular interest are alignment programs that permit gaps in the sequence. The Smith-Waterman is one type of algorithm that permits gaps in sequence alignments. See Meth. Mol. Biol. 70: 173-187 (1997). Also, the GAP program using the Needleman and Wunsch alignment method can be utilized to align sequences. See J. Mol. Biol. 48: 443-453 (1970).

Before the present invention is further described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs.

It must be noted that as used herein and in the appended claims, the singular forms "a," "an," and "the" include plural referents unless the context clearly dictates otherwise. Thus, for example, reference to "a stem cell" includes a plurality of such stem cells and reference to "the genome editing endonuclease" includes reference to one or more genome editing endonucleases and equivalents thereof known to those skilled in the art, and so forth. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination. All combinations of the embodiments pertaining to the invention are specifically embraced by the present invention and are disclosed herein just as if each and every combination was individually and explicitly disclosed. In addition, all subcombinations of the various embodiments and elements thereof are also specifically embraced by the present invention and are disclosed herein just as if each and every such sub-combination was individually and explicitly disclosed herein.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

### DETAILED DESCRIPTION

The present disclosure provides methods for gene editing in stem cells, as set out in the claims. The methods generally involve modifying the stem cells by increasing the level of an apoptosis regulator in the stem cells; and introducing into the modified stem cells a genome editing composition. Genome editing includes NHEJ and HDR. A genome-editing endonuclease generates a single- or double-strand break in a target genomic DNA, and the single- or double-strand break is repaired. Repair that occurs via NHEJ is sometimes referred to an "indel" (insertion or deletion); DNA repair via HDR is sometimes referred to as "gene correction" or "gene modification." In some cases, editing a target genomic DNA involves generating a substitution of one or more nucleotides in the target genomic DNA, generating an edited target genomic DNA. In some cases, editing a target genomic DNA involves deletion of one or more nucleotides from the target genomic DNA, generating an edited target genomic DNA. In some cases, editing a target genomic DNA involves insertion of one or more nucleotides from the target genomic DNA, generating an edited target genomic DNA.

The present disclosure provides a method of *ex vivo* editing a target genomic DNA in a mammalian stem cell, as set out in the claims.

In some cases, the apoptosis regulator is transiently overexpressed. For example, in some cases, the apoptosis regulator is overexpressed for a period of time of from about 1 hour to about 48 hours. For example, in some cases, the apoptosis regulator is overexpressed for a period of time of from about 1 hour to about 4 hours, from about 4 hours to about 8 hours, from about 8 hours to about 12 hours, from about 12 hours to about 16 hours, from about 16 hours to about 20 hours, from about 20 hours to about 24 hours, from about 24 hours to about 30 hours, from about 30 hours to about 36 hours, from about 36 hours to about 42 hours, or from about 42 hours to about 48 hours. In some cases, the apoptosis regulator is overexpressed for a period of time of from about 1 hour to about 72 hours. In some cases, the apoptosis regulator is overexpressed for a period of time of from about 48 hours to about 72 hours, *e.g.,* from about 48 hours to about 54 hours, from about 54 hours to about 60 hours, from about 60 hours to about 66 hours, or from about 66 hours to about 72 hours.

The apoptosis regulator is overexpressed such that the level of the apoptosis regulator in the modified stem cell is at least 25%, at least 50%, at least 75%, at least 2-fold, at least 2.5-fold, at least 5-fold, at least 10-fold, at least 25-fold, or more than 25-fold, higher than the background level of the apoptosis regulator in a control (unmodified) stem cell. For example, in some cases, the apoptosis regulator is overexpressed such that the level of the apoptosis regulator in the modified stem cell is from 25% to 50%, from 50% to 75%, from 75% to 2-fold, from 2-fold to 2.5-fold, from 2.5-fold to 5-fold, from 5-fold to 10-fold, from 10-fold to 25-fold, or from 25-fold to 50-fold, higher than the background level of the apoptosis regulator in a control (unmodified) stem cell.

Overexpressing an apoptosis regulator in a mammalian stem cell generates a modified mammalian stem cell. Thus, the step of contacting the mammalian stem cell with a genome editing composition, or with an RNP, occurs during a period of time in which the apoptosis regulator is overexpressed.

Overexpression of an apoptosis regulator in a mammalian stem cell can be achieved by any known method. In some cases, an apoptosis regulator is introduced in a stem cell as a polypeptide. In such cases, the apoptosis regulator can be modified with a heterologous polypeptide that facilitates entry into the cell. For example, a fusion protein comprising an apoptosis regulator and a fusion partner that facilitates entry into a stem cell can be used. Suitable fusion partners include, *e.g.,* a protein transduction domain (PTD; also referred to as a cell-penetrating peptide or CPP). Examples of PTDs include but are not limited to a minimal undecapeptide protein transduction domain (corresponding to residues 47-57 of HIV-1 TAT comprising YGRKKRRQRRR; SEQ ID NO: 1076); a polyarginine sequence comprising a number of arginines sufficient to direct entry into a cell *(e.g.,* 3, 4, 5, 6, 7, 8, 9, 10, or 10-50 arginines); a VP22 domain (Zender et al. (2002) Cancer Gene Ther. 9(6):489-96); an Drosophila Antennapedia protein transduction domain (Noguchi et al. (2003) Diabetes 52(7):1732-1737); a truncated human calcitonin peptide (Trehin et al. (2004) Pharm. Research 21:1248-1256); polylysine (Wender et al. (2000) Proc. Natl. Acad. Sci. USA 97:13003-13008); RRQRRTSKLMKR (SEQ ID NO:1077); Transportan GWTLNSAGYLLGKINLKALAALAKKIL (SEQ ID NO: 1078); KALAWEAKLAKALAKALAKHLAKALAKALKCEA (SEQ ID NO:1079); and RQIKIWFQNRRMKWKK (SEQ ID NO:1080). Exemplary PTDs include but are not limited to, YGRKKRRQRRR (SEQ ID NO:1081), RKKRRQRRR (SEQ ID NO: 1082); an arginine homopolymer of from 3 arginine residues to 50 arginine residues; Exemplary PTD domain amino acid sequences include, but are not limited to, any of the following: YGRKKRRQRRR (SEQ ID NO:1083); RKKRRQRR (SEQ ID NO:1084); YARAAARQARA (SEQ ID NO: 1085); THRLPRRRRRR (SEQ ID NO: 1086); and GGRRARRRRRR (SEQ ID NO:1087). In some embodiments, the PTD is an activatable CPP (ACPP) (Aguilera *et al.* (2009) *Integr Biol (Camb)* June; 1(5-6): 371-381). ACPPs comprise a polycationic CPP *(e.g.,* Arg9 or "R9") connected via a cleavable linker to a matching polyanion *(e.g.,* Glu9 or "E9"), which reduces the net charge to nearly zero and thereby inhibits adhesion and uptake into cells. Upon cleavage of the linker, the polyanion is released, locally unmasking the polyarginine and its inherent adhesiveness, thus "activating" the ACPP to traverse the membrane.

In some cases, an mRNA comprising a nucleotide sequence encoding the apoptosis regulator is introduced into a mammalian stem cell, where the mRNA enters the stem cell and the apoptosis regulator-encoding mRNA is translated.

As another example, in some cases, a recombinant expression vector comprising a nucleotide sequence encoding an apoptosis regulator is introduced into a mammalian stem cell, where the apoptosis regulator-encoding nucleotide sequence is expressed in the stem cell. Suitable expression vectors that can be used to generate a recombinant expression vector comprising a nucleotide sequence encoding an apoptosis regulator are known in the art; suitable examples include adeno-associated virus vectors, lentivirus vectors, retroviral vectors, herpes simplex virus vectors, and the like. A nucleotide sequence encoding an apoptosis regulator can be operably linked to a transcriptional control element(s), *e.g.,* a promoter, where the promoter is active in a stem cell. In some cases, the promoter is a constitutive promoter. In some cases, the promoter is an inducible promoter.

Overexpression (*e.g.*, transient overexpression) of an apoptosis regulator in a stem cell increases the survival of the stem cell when the stem cell is contacted with a genome editing composition (*e.g.*, a composition comprising a genome editing nuclease; a composition comprising a genome editing nuclease and a donor polynucleotide; a composition comprising an RNA-guided genome editing nuclease and a guide RNA; a composition comprising an RNA-guided genome editing nuclease, a guide RNA, and a donor polynucleotide; *etc.,* as described below). For example, overexpression *(e.g.,* transient overexpression) of an apoptosis regulator in a stem cell increases the survival of the stem cell when the stem cell is contacted with a genome editing composition by at least 10%, at least 15%, at least 20%, at least 25%, at least 50%, at least 2-fold, at least 5-fold, at least 10-fold, at least 20-fold, at least 50-fold, or more than 50-fold, compared with the survival of a control stem cell that does not overexpress the apoptosis regulator, and is contacted with the genome editing composition. In some cases, overexpression (*e.g.*, transient overexpression) of an apoptosis regulator in a population of stem cells increases the percent of the stem cells that survive genome editing *(e.g.,* that survive contact with a genome editing composition). For example, in some cases, overexpression (*e.g.*, transient overexpression) of an apoptosis regulator in a population of stem cells increases the percent of the stem cells that survive genome editing *(e.g.,* that survive contact with a genome editing composition) by at least 10%, at least 15%, at least 20%, at least 25%, at least 50%, at least 2-fold, at least 3-fold, at least 4-fold, or at least 5-fold, compared with the percent of control stem cells that survive genome editing *(e.g.,* that survive contact with a genome editing composition), where the control stem cells do not overexpress the apoptosis regulator. As another example, in some cases, at least 40%, at least 50%, at least 60%, at least 70%, at least 75%, at least 80%, at least 85%, at least 90%, or at least 95%, of a stem cell population that overexpresses (*e.g*., transiently overexpresses) an apoptosis regulator survives genome editing (*e.g*., survives contact with a genome editing composition). Thus, the efficiency of genome editing of a stem cell is increased when the stem cell overexpresses an apoptosis regulator.

### Apoptosis regulators

Suitable apoptosis regulators inhibit apoptosis in a stem cell. The apoptosis regulator used in the claimed methods is Bc1-2.

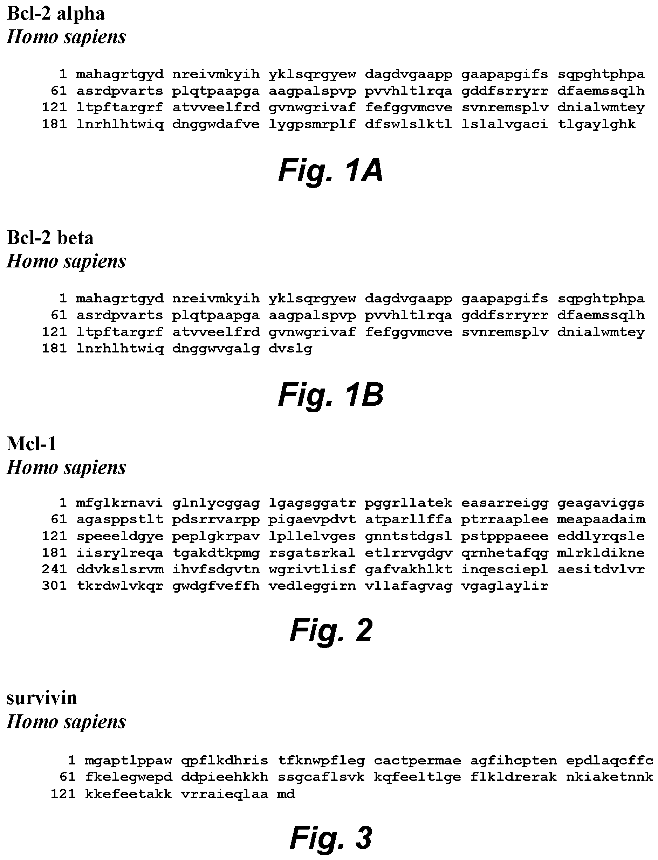

In some cases, the apoptosis regulator is a Bcl-2 polypeptide comprising an amino acid sequence having at least 85%, at least 90%, at least 95%, at least 98%, at least 99%, or 100%, amino acid sequence identity to the amino acid sequence depicted in Fig. 1A.

In some cases, the apoptosis regulator is a Bcl-2 polypeptide comprising an amino acid sequence having at least 85%, at least 90%, at least 95%, at least 98%, at least 99%, or 100%, amino acid sequence identity to the amino acid sequence depicted in Fig. 1B.

### Stem cells

The present disclosure provides methods of editing a target genomic DNA of a mammalian stem cell, which is a pluripotent stem cell or an adult stem cell; and/or a hematopoietic stem cell, a neural stem cell, a mesenchymal stem cell, or an induced pluripotent stem cell.

Hematopoietic stem cells (HSCs) are mesoderm-derived cells that can be isolated from bone marrow, blood, cord blood, fetal liver and yolk sac. HSCs are characterized as CD34⁺ and CD3⁻. HSCs can repopulate the erythroid, neutrophil-macrophage, megakaryocyte and lymphoid hematopoietic cell lineages *in vivo. In vitro,* HSCs can be induced to undergo at least some self-renewing cell divisions and can be induced to differentiate to the same lineages as is seen *in vivo.* As such, HSCs can be induced to differentiate into one or more of erythroid cells, megakaryocytes, neutrophils, macrophages, and lymphoid cells.

Neural stem cells (NSCs) are capable of differentiating into neurons, and glia (including oligodendrocytes, and astrocytes). A neural stem cell is a multipotent stem cell which is capable of multiple divisions, and under specific conditions can produce daughter cells which are neural stem cells, or neural progenitor cells that can be neuroblasts or glioblasts, *e.g.,* cells committed to become one or more types of neurons and glial cells respectively. Methods of obtaining NSCs are known in the art.

Mesenchymal stem cells (MSC), originally derived from the embryonal mesoderm and isolated from adult bone marrow, can differentiate to form muscle, bone, cartilage, fat, marrow stroma, and tendon. Methods of isolating MSC are known in the art; and any known method can be used to obtain MSC. See, *e.g.,* U.S. Pat. No. 5,736,396, which describes isolation of human MSC.

An induced pluripotent stem (iPS) cells is a pluripotent stem cell induced from a somatic cell, *e.g.,* a differentiated somatic cell. iPS cells are capable of self-renewal and differentiation into cell fate-committed stem cells, including neural stem cells, as well as various types of mature cells.

iPS cells can be generated from somatic cells, including skin fibroblasts, using, *e.g.*, known methods. iPS cells produce and express on their cell surface one or more of the following cell surface antigens: SSEA-3, SSEA-4, TRA-1-60, TRA-1-81, TRA-2-49/6E, and Nanog. In some cases, iPS cells produce and express on their cell surface SSEA-3, SSEA-4, TRA-1-60, TRA-1-81, TRA-2-49/6E, and Nanog. iPS cells express one or more of the following genes: Oct-3/4, Sox2, Nanog, GDF3, REX1, FGF4, ESG1, DPPA2, DPPA4, and hTERT. In some embodiments, an iPS cell expresses Oct-3/4, Sox2, Nanog, GDF3, REX1, FGF4, ESG1, DPPA2, DPPA4, and hTERT. Methods of generating iPS are known in the art, and any such method can be used to generate iPS. See, *e.g.,* Takahashi and Yamanaka (2006) Cell 126:663-676; Yamanaka et. al. (2007) Nature 448:313-7; Wernig et. al. (2007) Nature 448:318-24; Maherali (2007) Cell Stem Cell 1:55-70; Nakagawa et al. (2008) Nat. Biotechnol. 26:101; Takahashi et al. (2007) Cell 131:861; Takahashi et al. (2007) Nat. Protoc. 2:3081; and Okita et al. (2007 Nature 448:313.

iPS cells can be generated from somatic cells *(e.g.,* skin fibroblasts) by genetically modifying the somatic cells with one or more expression constructs encoding Oct-3/4 and Sox2. In some cases, somatic cells are genetically modified with one or more expression constructs comprising nucleotide sequences encoding Oct-3/4, Sox2, c-myc, and Klf4. In some embodiments, somatic cells are genetically modified with one or more expression constructs comprising nucleotide sequences encoding Oct-4, Sox2, Nanog, and LIN28.

In some cases, a target genomic DNA is the genomic DNA of a mammalian hematopoietic stem cell (HSC) *(e.g.,* mobilized peripheral blood (mPB) CD34(+) cells, bone marrow (BM) CD34(+) cells, and the like). In some cases, the HSC is a long-term (LT)-HSC. The HSC can be from any mammal, *e.g.,* a horse, dog, cat, goat, cow, pig, sheep, primate, non-human primate, or human. Suitable HSCs include, but are not limited to: mobilized peripheral blood (mPB) CD34(+) cells and bone marrow (BM) CD34(+) cells.

In some cases, the mammalian HSC *(e.g.,* human HSC) is a mobilized peripheral blood (mPB) CD34(+) cell or a bone marrow (BM) CD34(+) cell. In some cases, the mammalian HSC *(e.g.,* human HSC) is a long-term (LT)-HSC. In some cases, the mammalian HSC *(e.g.,* human HSC) is from an individual with a sickle cell disease or with X-linked hyper IgM syndrome. In some cases, the mammalian HSC *(e.g.,* human HSC) is from an individual with X-linked gammaglobulinemia.

In some cases, the HSC *(e.g.,* a mobilized peripheral blood (mPB) CD34(+) cell, a bone marrow (BM) CD34(+) cell, *etc.)* into which the subject compositions are introduced is ex vivo. For example, in some cases, the HSC into which the subject compositions are introduced is isolated *(e.g.,* in culture), and in some cases the HSC is a primary cell *(e.g.,* a mobilized peripheral blood (mPB) CD34(+) cell, a bone marrow (BM) CD34(+) cell, *etc.)* that was isolated/harvested from an individual *(e.g.,* an individual with a disease such as sickle cell disease or X-linked hyper IgM syndrome). In some cases, the individual from whom the HSC *(e.g.,* a mobilized peripheral blood (mPB) CD34(+) cell, a bone marrow (BM) CD34(+) cell, *etc.)* is isolated has X-Linked Hyper-IgM Syndrome *(e.g.,* caused by a mutation in the gene CD40 Ligand (CD40L)). In some cases, the individual from whom the HSC *(e.g.,* a mobilized peripheral blood (mPB) CD34(+) cell, a bone marrow (BM) CD34(+) cell, *etc.)* is isolated has Sickle cell disease *(e.g.,* caused by a mutation in the beta-globin gene (HBB)). In some cases, the individual from whom the HSC *(e.g.,* a mobilized peripheral blood (mPB) CD34(+) cell, a bone marrow (BM) CD34(+) cell, *etc.)* is isolated has X-Linked agammaglobulinemia (XLA) *(e.g.,* caused by a mutation in the Bruton tyrosine kinase (BTK) gene).

HSCs can be obtained from several different sources including bone marrow, mobilized peripheral blood, and umbilical cord blood. Methods of harvesting HSCs *(e.g.,* mobilized peripheral blood (mPB) CD34(+) cells, bone marrow (BM) CD34(+) cells, and the like) from an individual will be known to one of ordinary skill in the art and any convenient method can be used. For example, G-CSF can be used to mobilize HSCs, which can be harvested as mobilized peripheral blood (mPB) CD34(+) cells. For example, harvesting HSCs from bone marrow (BM) can be a one-time, single-day procedure whereas harvesting HSCs from mobilized peripheral blood (mPB) can extend over the stem cell mobilization period *(e.g.,* 4 to 5 days) and the harvesting period *(e.g.,* 1 to 3 days).

In some cases, granulocyte colony-stimulating factor (G-CSF) is used as an HSC mobilizing agent (*e.g., i.e.*, an agent to increase the level of circulating HSCs). For example, the administration of G-CSF daily for 4 to 6 days can results in a 10- to 30-fold increase in the number of circulating HSCs and G-CSF-mobilized HSCs collected by apheresis can be used for various purposes (*e.g.*, transplantation, immune therapy, treatment of cardiac ischemia, cancer treatment, *etc.).* Any convenient HSC mobilizing agent can be used and several such agents will be known to one of ordinary skill in the art. Suitable HSC mobilization agents include, *e.g.*, granulocyte colony stimulating factor (G-CSF), granulocyte-macrophage colony stimulating factor (GM-CSF), GRO-β (CXCL2), an N-terminal 4-amino acid truncated form of GRO-β, pegfilgrastim, AMD-3100 (1,1'-[1,4-Phenylenebis(methylene)]bis [1,4,8,11-tetraazacyclotetradecane]), and the like.

### Genome Editing Composition

A genome editing composition is a composition that includes a genome editing nuclease that is (or can be) targeted to a desired sequence within a target genome. A genome editing nuclease is an endonuclease capable of cleaving the phosphodiester bond within a polynucleotide chain at a designated specific site within a selected genomic target DNA *(e.g.,* causing a double-stranded break (DSB)) without damaging the bases. In some embodiments, the genome editing nuclease binds a native or endogenous recognition sequence. In some embodiments, the genome editing nuclease is a modified endonuclease that binds a non-native or exogenous recognition sequence and does not bind a native or endogenous recognition sequence.

Examples of suitable genome editing nucleases include but are not limited to zinc finger nucleases, TAL-effector DNA binding domain-nuclease fusion proteins (transcription activator-like effector nucleases (TALENs)), and CRISPR/Cas endonucleases *(e.g.,* class 2 CRISPR/Cas endonucleases such as a type II, type V, or type VI CRISPR/Cas endonucleases). Thus, in some embodiments, a genome editing composition can include one or more genome editing nucleases selected from: a zinc finger nuclease, a TAL-effector DNA binding domain-nuclease fusion protein (TALEN), and a CRISPR/Cas endonuclease *(e.g.,* a class 2 CRISPR/Cas endonuclease such as a type II, type V, or type VI CRISPR/Cas endonuclease). In some cases, a genome editing composition includes a zinc finger nuclease or a TALEN. In some cases, a genome editing composition includes a class 2 CRISPR/Cas endonuclease. In some cases, a genome editing composition includes a class 2 type II CRISPR/Cas endonuclease *(e.g.,* a Cas9 protein). In some cases, a genome editing composition includes a class 2 type V CRISPR/Cas endonuclease *(e.g.,* a Cpfl protein, a C2c1 protein, or a C2c3 protein). In some cases, a genome editing composition includes a class 2 type VI CRISPR/Cas endonuclease *(e.g.,* a C2c2 protein).

As described in more detail below, a CRISPR/Cas endonuclease interacts with (binds to) a corresponding guide RNA to form a ribonucleoprotein (RNP) complex that is targeted to a particular site in a target genome via base pairing between the guide RNA and a target sequence within the target genome. A guide RNA includes a nucleotide sequence (a guide sequence) that is complementary to a sequence (the target site) of a target nucleic acid. Thus, when a subject genome editing composition includes a CRISPR/Cas endonuclease *(e.g.,* a class 2 CRISPR/Cas endonuclease), it must also include a corresponding guide RNA when being used in a method to cleave a target DNA. However, because the guide RNA can be readily modified in order to target any desired sequence within a target genome, in some cases, a composition includes only the CRISPR/Cas endonuclease (or a nucleic acid encoding the CRISPR/Cas endonuclease) until a user adds the desired corresponding guide RNA (or a nucleic acid encoding the corresponding guide RNA).

The components of a subject genome editing composition can be delivered (introduced into a stem cell) as DNA, RNA, or protein. For example, when the composition includes a class 2 CRISPR/Cas endonuclease *(e.g.,* Cas9, Cpfl, *etc.)* and a corresponding guide RNA *(e.g.,* a Cas9 guide RNA, a Cpfl guide RNA, *etc.),* the endonuclease and guide RNA can be delivered (introduced into the cell) as an RNP complex (*i.e.,* a pre-assembled complex of the CRISPR/Cas endonuclease and the corresponding CRISPR/Cas guide RNA). Thus, a class 2 CRISPR/Cas endonuclease can be introduced into a cell as a protein. Alternatively, a class 2 CRISPR/Cas endonuclease can be introduced into a cell as a nucleic acid (DNA and/or RNA) encoding the endonuclease. A CRISPR/Cas guide RNA can be introduced into a cell as RNA, or as DNA encoding the guide RNA. Likewise, a zinc finger nuclease and/or a TALEN can be introduced into a cell as a protein or alternatively as a nucleic acid (DNA and/or RNA) encoding the protein.

In some cases, a genome editing nuclease is a fusion protein that is fused to a heterologous polypeptide (also referred to as a "fusion partner"). In some cases, a genome editing nuclease is fused to an amino acid sequence (a fusion partner) that provides for subcellular localization, *i.e.*, the fusion partner is a subcellular localization sequence *(e.g.,* one or more nuclear localization signals (NLSs) for targeting to the nucleus, two or more NLSs, three or more NLSs, *etc.).* In some embodiments, a genome editing nuclease is fused to an amino acid sequence (a fusion partner) that provides a tag *(i.e.,* the fusion partner is a detectable label) for ease of tracking and/or purification *(e.g.,* a fluorescent protein, *e.g.,* green fluorescent protein (GFP), YFP, RFP, CFP, mCherry, tdTomato, and the like; a histidine tag, *e.g.,* a 6XHis tag; a hemagglutinin (HA) tag; a FLAG tag; a Myc tag; and the like). In some embodiments, the fusion partner can provide for increased or decreased stability (*i.e.,* the fusion partner can be a stability control peptide, *e.g.,* a degron, which in some cases is controllable *(e.g.,* a temperature sensitive or drug controllable degron sequence).

In some cases, a genome editing nuclease is conjugated (*e.g*., fused) to a polypeptide permeant domain to promote uptake by the cell (*i.e.,* the fusion partner promotes uptake by a cell). A number of permeant domains are known in the art and may be used, including peptides, peptidomimetics, and non-peptide carriers. For example, a permeant peptide may be derived from the third alpha helix of Drosophila melanogaster transcription factor Antennapaedia, referred to as penetratin, which comprises the amino acid sequence RQIKIWFQNRRMKWKK (SEQ ID NO:1080). As another example, the permeant peptide can comprise the HIV-1 tat basic region amino acid sequence, which may include, for example, amino acids 49-57 of naturally-occurring tat protein. Other permeant domains include poly-arginine motifs, for example, the region of amino acids 34-56 of HIV-1 rev protein, nona-arginine, octa-arginine, and the like. (See, for example, Futaki et al. (2003) Curr Protein Pept Sci. 2003 Apr; 4(2): 87-9 and 446; and Wender et al. (2000) Proc. Natl. Acad. Sci. U.S.A 2000 Nov. 21; 97(24):13003-8; published U.S. Patent applications 20030220334; 20030083256; 20030032593; and 20030022831). The nona-arginine (R9) sequence is one of the more efficient PTDs that have been characterized (Wender *et al.* 2000; Uemura *et al.* 2002). The site at which the fusion is made may be selected in order to optimize the biological activity, secretion or binding characteristics of the polypeptide. The optimal site can be determined by routine experimentation.

In some cases, a genome editing nuclease includes a "Protein Transduction Domain" or PTD (also known as a CPP - cell penetrating peptide), which refers to a polypeptide, polynucleotide, carbohydrate, or organic or inorganic compound that facilitates traversing a lipid bilayer, micelle, cell membrane, organelle membrane, or vesicle membrane. A PTD attached to another molecule, which can range from a small polar molecule to a large macromolecule and/or a nanoparticle, facilitates the molecule traversing a membrane, for example going from extracellular space to intracellular space, or cytosol to within an organelle. In some embodiments, a PTD is covalently linked to the amino terminus a polypeptide *(e.g.,* a genome editing nuclease, *e.g.,* a Cas9 protein). In some embodiments, a PTD is covalently linked to the carboxyl terminus of a polypeptide *(e.g.,* a genome editing nuclease, *e.g.,* a Cas9 protein). In some cases, the PTD is inserted internally in the genome editing nuclease *(e.g.,* Cas9 protein) *(i.e.,* is not at the N- or C-terminus of the genome editing nuclease). In some cases, a subject genome editing nuclease *(e.g.,* Cas9 protein) includes (is conjugated to, is fused to) one or more PTDs *(e.g.,* two or more, three or more, four or more PTDs). In some cases a PTD includes a nuclear localization signal (NLS) *(e.g.,* in some cases 2 or more, 3 or more, 4 or more, or 5 or more NLSs).

In some cases, a genome editing nuclease *(e.g.,* Cas9 protein) includes one or more NLSs *(e.g.,* 2 or more, 3 or more, 4 or more, or 5 or more NLSs). In some embodiments, a PTD is covalently linked to a nucleic acid *(e.g.,* a CRISPR/Cas guide RNA, a polynucleotide encoding a CRISPR/Cas guide RNA, a polynucleotide encoding a class 2 CRISPR/Cas endonuclease such as a Cas9 protein or a type V or type VI CRISPR/Cas protein, *etc.).* Examples of PTDs include but are not limited to a minimal undecapeptide protein transduction domain (corresponding to residues 47-57 of HIV-1 TAT comprising YGRKKRRQRRR; SEQ ID NO:1076); a polyarginine sequence comprising a number of arginines sufficient to direct entry into a cell *(e.g.,* 3, 4, 5, 6, 7, 8, 9, 10, or 10-50 arginines); a VP22 domain (Zender et al. (2002) Cancer Gene Ther. 9(6):489-96); an Drosophila Antennapedia protein transduction domain (Noguchi et al. (2003) Diabetes 52(7):1732-1737); a truncated human calcitonin peptide (Trehin et al. (2004) Pharm. Research 21:1248-1256); polylysine (Wender et al. (2000) Proc. Natl. Acad. Sci. USA 97:13003-13008); RRQRRTSKLMKR (SEQ ID NO:1077); Transportan GWTLNSAGYLLGKINLKALAALAKKIL (SEQ ID NO:1078); KALAWEAKLAKALAKALAKHLAKALAKALKCEA (SEQ ID NO:1079); and RQIKIWFQNRRMKWKK (SEQ ID NO:1080). Exemplary PTDs include but are not limited to, YGRKKRRQRRR (SEQ ID NO:1081), RKKRRQRRR (SEQ ID NO:1082); an arginine homopolymer of from 3 arginine residues to 50 arginine residues; Exemplary PTD domain amino acid sequences include, but are not limited to, any of the following: YGRKKRRQRRR (SEQ ID NO:1083); RKKRRQRR (SEQ ID NO:1084); YARAAARQARA (SEQ ID NO: 1085); THRLPRRRRRR (SEQ ID NO: 1086); and GGRRARRRRRR (SEQ ID NO:1087). In some embodiments, the PTD is an activatable CPP (ACPP) (Aguilera *et al.* (2009) *Integr Biol (Camb)* June; 1(5-6): 371-381). ACPPs comprise a polycationic CPP *(e.g.,* Arg9 or "R9") connected via a cleavable linker to a matching polyanion (*e.g.*, Glu9 or "E9"), which reduces the net charge to nearly zero and thereby inhibits adhesion and uptake into cells. Upon cleavage of the linker, the polyanion is released, locally unmasking the polyarginine and its inherent adhesiveness, thus "activating" the ACPP to traverse the membrane.

A genome editing nuclease *(e.g.,* Cas9 protein) can have multiple (1 or more, 2 or more, 3 or more, *etc.)* fusion partners in any combination of the above. As an illustrative example, a genome editing nuclease *(e.g.,* Cas9 protein) can have a fusion partner that provides for tagging *(e.g.,* GFP), and can also have a subcellular localization sequence *(e.g.,* one or more NLSs). In some cases, such a fusion protein might also have a tag for ease of tracking and/or purification *(e.g.,* a histidine tag, *e.g.,* a 6XHis (His-His-His-His-His-His, SEQ ID NO:1149) tag; a hemagglutinin (HA) tag; a FLAG tag; a Myc tag; and the like). As another illustrative example, genome editing nuclease *(e.g.,* Cas9 protein) can have one or more NLSs *(e.g.,* two or more, three or more, four or more, five or more, 1, 2, 3, 4, or 5 NLSs). In some cases a fusion partner (or multiple fusion partners, *e.g.,* 1, 2, 3, 4, or 5 NLSs) *(e.g.,* an NLS, a tag, a fusion partner providing an activity, *etc.)* is located at or near the C-terminus of the genome editing nuclease *(e.g.,* Cas9 protein). In some cases a fusion partner (or multiple fusion partners, *e.g.,* 1, 2, 3, 4, or 5 NLSs) *(e.g.,* an NLS, a tag, a fusion partner providing an activity, *etc.)* is located at the N-terminus of the genome editing nuclease *(e.g.,* Cas9 protein). In some cases the genome editing nuclease *(e.g.,* Cas9 protein) has a fusion partner (or multiple fusion partners, *e.g.,* 1, 2, 3, 4, or 5 NLSs)(e.g., an NLS, a tag, a fusion partner providing an activity, *etc.)* at both the N-terminus and C-terminus.

### Zinc Finger nucleases (ZFNs)

In some cases, a genome editing nuclease used in a method of the present disclosure, or included in a genome editing composition, is a zinc-finger nuclease (ZFN). ZFNs are engineered double-strand break inducing proteins comprised of a zinc finger DNA binding domain and a double strand break inducing agent domain. Engineered ZFNs consist of two zinc finger arrays (ZFAs), each of which is fused to a single subunit of a non-specific endonuclease, such as the nuclease domain from the FokI enzyme, which becomes active upon dimerization. Typically, a single ZFA consists of 3 or 4 zinc finger domains, each of which is designed to recognize a specific nucleotide triplet (GGC, GAT, *etc.).* Thus, ZFNs composed of two "3-finger" ZFAs are capable of recognizing an 18 base pair target site; an 18 base pair recognition sequence is generally unique, even within large genomes such as those of humans and plants. By directing the co-localization and dimerization of two FokI nuclease monomers, ZFNs generate a functional site-specific endonuclease that creates a double-stranded break (DSB) in DNA at the targeted locus. Zinc-finger endonucleases suitable for genome editing are known in the art. See, *e.g.,* Hoban et al. (2015) Blood 125:2597.

Useful zinc-finger nucleases include those that are known and those that are engineered to have specificity for one or more desired target sites (TS). Zinc finger domains are amenable for designing polypeptides which specifically bind a selected polynucleotide recognition sequence, for example, within the target site of the host cell genome. ZFNs consist of an engineered DNA-binding zinc finger domain linked to a non-specific endonuclease domain, for example nuclease domain from a Type IIs endonuclease such as HO or FokI. Alternatively, engineered zinc finger DNA binding domains can be fused to other double-strand break inducing agents or derivatives thereof that retain DNA nicking/cleaving activity. For example, this type of fusion can be used to direct the double-strand break inducing agent to a different target site, to alter the location of the nick or cleavage site, to direct the inducing agent to a shorter target site, or to direct the inducing agent to a longer target site. In some examples a zinc finger DNA binding domain is fused to a site-specific recombinase, transposase, or a derivative thereof that retains DNA nicking and/or cleaving activity. Additional functionalities can be fused to the zinc-finger binding domain, including transcriptional activator domains, transcription repressor domains, and methylases. In some embodiments, dimerization of nuclease domain is required for cleavage activity.

Each zinc finger recognizes three consecutive base pairs in the target DNA. For example, a 3 finger domain recognized a sequence of 9 contiguous nucleotides, with a dimerization requirement of the nuclease, two sets of zinc finger triplets are used to bind a 18 nucleotide recognition sequence. Useful designer zinc finger modules include those that recognize various GNN and ANN triplets (Dreier, et al., (2001) J Biol Chem 276:29466-78; Dreier, et al., (2000) J Mol Biol 303:489-502; Liu, et al., (2002) J Biol Chem 277:3850-6), as well as those that recognize various CNN or TNN triplets (Dreier, et al., (2005) J Biol Chem 280:35588-97; Jamieson, et al., (2003) Nature Rev Drug Discov 2:361-8). See also, Durai, et al., (2005) Nucleic Acids Res 33:5978-90; Segal, (2002) Methods 26:76-83; Porteus and Carroll, (2005) Nat Biotechnol 23:967-73; Pabo, et al., (2001) Ann Rev Biochem 70:313-40; Wolfe, et al., (2000) Ann Rev Biophys Biomol Struct 29:183-212; Segal and Barbas, (2001) Curr Opin Biotechnol 12:632-7; Segal, et al., (2003) Biochemistry 42:2137-48; Beerli and Barbas, (2002) Nat Biotechnol 20:135-41; Carroll, et al., (2006) Nature Protocols 1:1329; Ordiz, et al., (2002) Proc Natl Acad Sci USA 99:13290-5; Guan, et al., (2002) Proc Natl Acad Sci USA 99:13296-301; WO2002099084; WO00/42219; WO02/42459; WO2003062455; US20030059767; US Patent Publication Number 2003/0108880; US Patent Publication Number 2014/0093913; U.S. Pat. Nos. 6,140,466, 6,511,808 and 6,453,242. Useful zinc-finger nucleases also include those described in WO03/080809; WO05/014791; WO05/084190; WO08/021207; WO09/042186; WO09/054985; and WO10/065123.

If the genome editing endonuclease to be utilized is a zinc finger nuclease, optimal target sites may be selected using a number of publicly available online resources. See, *e.g.,* Reyon *et al.,* BMC Genomics 12:83 (2011). For example, Oligomerized Pool Engineering (OPEN) is a highly robust and publicly available protocol for engineering zinc finger arrays with high specificity and *in vivo* functionality, and has been successfully used to generate ZFNs that function efficiently in plants, zebrafish, and human somatic and pluripotent stem cells. OPEN is a selection-based method in which a pre-constructed randomized pool of candidate ZFAs is screened to identify those with high affinity and specificity for a desired target sequence. ZFNGenome is a GBrowse-based tool for identifying and visualizing potential target sites for OPEN-generated ZFNs. ZFNGenome provides a compendium of potential ZFN target sites in sequenced and annotated genomes of model organisms. ZFNGenome currently includes a total of more than 11.6 million potential ZFN target sites, mapped within the fully sequenced genomes of seven model organisms; *Saccharomyces cerevisiae, Chlamydomonas reinhardtii, Arabidopsis thaliana, Drosophila melanogaster, Danio rerio, Caenorhabditis elegans,* and *Homo sapiens.* Additional model organisms, including three plant species; *Glycine max* (soybean), *Oryza sativa* (rice), *Zea mays* (maize), and three animal species *Tribolium castaneum* (red flour beetle), *Mus musculus* (mouse), *Rattus norvegicus* (brown rat) can also be used. ZFNGenome provides information about each potential ZFN target site, including its chromosomal location and position relative to transcription initiation site(s). Users can query ZFNGenome using several different criteria *(e.g.,* gene ID, transcript ID, target site sequence).

For more information on ZFNs, refer to U.S. Patent No. 8,685,737.

### TALENs

In some embodiments, a subject genome editing nuclease is a TAL-effector DNA binding domain-nuclease fusion protein (TALEN). A TAL effector comprises a DNA binding domain that interacts with DNA in a sequence-specific manner through one or more tandem repeat domains. The repeated sequence typically comprises 34 amino acids, and the repeats are typically 91-100% homologous with each other. Polymorphism of the repeats is usually located at positions 12 and 13, and there appears to be a one-to-one correspondence between the identity of repeat variable-diresidues at positions 12 and 13 with the identity of the contiguous nucleotides in the TAL-effector's target sequence.

The TAL-effector DNA binding domain can be engineered to bind to a desired target sequence, and fused to a nuclease domain, *e.g*., from a type II restriction endonuclease, typically a nonspecific cleavage domain from a type II restriction endonuclease such as FokI (see *e.g.,* Kim et al. (1996) Proc. Natl. Acad. Sci. USA 93:1156-1160). Other useful endonucleases may include, for example, HhaI, HindIII, Nod, BbvCI, EcoRI, BglI, and AlwI. Thus, in some embodiments, a TALEN comprises a TAL effector domain comprising a plurality of TAL effector repeat sequences that, in combination, bind to a specific nucleotide sequence in the target DNA sequence, such that the TALEN cleaves the target DNA within or adjacent to the specific nucleotide sequence. Suitable TALENs include those described in WO10/079430 and U.S. Patent Application Publication No. 2011/0145940.

In some embodiments, the TAL effector domain that binds to a specific nucleotide sequence within the target DNA can comprise 10 or more DNA binding repeats, and in some cases 15 or more DNA binding repeats. In some embodiments, each DNA binding repeat comprises a repeat variable-diresidue (RVD) that determines recognition of a base pair in the target DNA sequence, wherein each DNA binding repeat is responsible for recognizing one base pair in the target DNA sequence, and wherein the RVD comprises one or more of: HD for recognizing C; NG for recognizing T; NI for recognizing A; NN for recognizing G or A; NS for recognizing A or C or G or T; N^{∗} for recognizing C or T, where ^{∗} represents a gap in the second position of the RVD; HG for recognizing T; H^{∗} for recognizing T, where ^{∗} represents a gap in the second position of the RVD; IG for recognizing T; NK for recognizing G; HA for recognizing C; ND for recognizing C; HI for recognizing C; HN for recognizing G; NA for recognizing G; SN for recognizing G or A; and YG for recognizing T.

If the genome editing endonuclease to be utilized is a TALEN, in some embodiments, optimal target sites may be selected in accordance with the methods described by Sanjana et al., Nature Protocols, 7:171-192 (2012). In brief, TALENs function as dimers, and a pair of TALENs, referred to as the left and right TALENs, target sequences on opposite strands of DNA. TALENs can be engineered as a fusion of the TALE DNA-binding domain and a monomeric FokI catalytic domain. To facilitate FokI dimerization, the left and right TALEN target sites can be chosen with a spacing of approximately 14-20 bases. Therefore, for a pair of TALENs, each targeting 20-bp sequences, an optimal target site can have the form 5'-TN¹⁹N¹⁴⁻²⁰N¹⁹A-3', where the left TALEN targets 5'-TN¹⁹-3' and the right TALEN targets the antisense strand of 5'-N¹⁹A-3' (N=A, G, T or C).

For more information on TALENs, refer to U.S. Patent No. 8,685,737.

### Class 2 CRISPR/Cas endonucleases

RNA-mediated adaptive immune systems in bacteria and archaea rely on Clustered Regularly Interspaced Short Palindromic Repeat (CRISPR) genomic loci and CRISPR-associated (Cas) proteins that function together to provide protection from invading viruses and plasmids. In some embodiments, a genome editing nuclease of a genome editing composition of the present disclosure is a class 2 CRISPR/Cas endonuclease. Thus in some cases, a subject genome editing composition includes a class 2 CRISPR/Cas endonuclease (or a nucleic encoding the endonuclease). In class 2 CRISPR systems, the functions of the effector complex *(e.g.,* the cleavage of target DNA) are carried out by a single endonuclease *(e.g.,* see Zetsche et al, Cell. 2015 Oct 22;163(3):759-71; Makarova et al, Nat Rev Microbiol. 2015 Nov;13(11):722-36; and Shmakov et al., Mol Cell. 2015 Nov 5;60(3):385-97). As such, the term "class 2 CRISPR/Cas protein" is used herein to encompass the endonuclease (the target nucleic acid cleaving protein) from class 2 CRISPR systems. Thus, the term "class 2 CRISPR/Cas endonuclease" as used herein encompasses type II CRISPR/Cas proteins *(e.g.,* Cas9), type V CRISPR/Cas proteins *(e.g.,* Cpfl, C2c1, C2C3), and type VI CRISPR/Cas proteins *(e.g.,* C2c2). To date, class 2 CRISPR/Cas proteins encompass type II, type V, and type VI CRISPR/Cas proteins, but the term is also meant to encompass any class 2 CRISPR/Cas protein suitable for binding to a corresponding guide RNA and forming an RNP complex.

### Type II CRISPR/Cas endonucleases (e.g., Cas 9)

In natural Type II CRISPR/Cas systems, Cas9 functions as an RNA-guided endonuclease that uses a dual-guide RNA having a crRNA and *trans*-activating crRNA (tracrRNA) for target recognition and cleavage by a mechanism involving two nuclease active sites in Cas9 that together generate double-stranded DNA breaks (DSBs), or can individually generate single-stranded DNA breaks (SSBs). The Type II CRISPR endonuclease Cas9 and engineered dual- (dgRNA) or single guide RNA (sgRNA) form a ribonucleoprotein (RNP) complex that can be targeted to a desired DNA sequence. Guided by a dual-RNA complex or a chimeric single-guide RNA, Cas9 generates site-specific DSBs or SSBs within double-stranded DNA (dsDNA) target nucleic acids, which are repaired either by non-homologous end joining (NHEJ) or homology-directed recombination (HDR).

As noted above, in some cases, a genome editing composition of the present disclosure includes a type II CRISPR/Cas endonuclease. A type II CRISPR/Cas endonuclease is a type of class 2 CRISPR/Cas endonuclease. In some cases, the type II CRISPR/Cas endonuclease is a Cas9 protein. A Cas9 protein forms a complex with a Cas9 guide RNA. The guide RNA provides target specificity to a Cas9-guide RNA complex by having a nucleotide sequence (a guide sequence) that is complementary to a sequence (the target site) of a target nucleic acid (as described elsewhere herein). The Cas9 protein of the complex provides the site-specific activity. In other words, the Cas9 protein is guided to a target site *(e.g.,* stabilized at a target site) within a target nucleic acid sequence *(e.g.,* a chromosomal sequence or an extrachromosomal sequence, *e.g.,* an episomal sequence, a minicircle sequence, a mitochondrial sequence, a chloroplast sequence, *etc.)* by virtue of its association with the protein-binding segment of the Cas9 guide RNA

A Cas9 protein can bind and/or modify *(e.g.,* cleave, nick, methylate, demethylate, *etc.)* a target nucleic acid and/or a polypeptide associated with target nucleic acid (*e.g.*, methylation or acetylation of a histone tail)(e.g., when the Cas9 protein includes a fusion partner with an activity). In some cases, the Cas9 protein is a naturally-occurring protein (*e.g.*, naturally occurs in bacterial and/or archaeal cells). In other cases, the Cas9 protein is not a naturally-occurring polypeptide *(e.g.,* the Cas9 protein is a variant Cas9 protein, a chimeric protein, and the like)

Examples of suitable Cas9 proteins include, but are not limited to, those set forth in SEQ ID NOs: 5-816. Naturally occurring Cas9 proteins bind a Cas9 guide RNA, are thereby directed to a specific sequence within a target nucleic acid (a target site), and cleave the target nucleic acid *(e.g.,* cleave dsDNA to generate a double strand break, cleave ssDNA, cleave ssRNA, *etc.).* A chimeric Cas9 protein is a fusion protein comprising a Cas9 polypeptide that is fused to a heterologous protein (referred to as a fusion partner), where the heterologous protein provides an activity *(e.g.,* one that is not provided by the Cas9 protein). The fusion partner can provide an activity, *e.g.,* enzymatic activity *(e.g.,* nuclease activity, activity for DNA and/or RNA methylation, activity for DNA and/or RNA cleavage, activity for histone acetylation, activity for histone methylation, activity for RNA modification, activity for RNA-binding, activity for RNA splicing *etc.).* In some cases a portion of the Cas9 protein *(e.g.,* the RuvC domain and/or the HNH domain) exhibits reduced nuclease activity relative to the corresponding portion of a wild type Cas9 protein *(e.g.,* in some cases the Cas9 protein is a nickase). In some cases, the Cas9 protein is enzymatically inactive, or has reduced enzymatic activity relative to a wild-type Cas9 protein (*e.g.*, relative to *Streptococcus pyogenes* Cas9).

Assays to determine whether given protein interacts with a Cas9 guide RNA can be any convenient binding assay that tests for binding between a protein and a nucleic acid. Suitable binding assays *(e.g.,* gel shift assays) will be known to one of ordinary skill in the art *(e.g.,* assays that include adding a Cas9 guide RNA and a protein to a target nucleic acid).

Assays to determine whether a protein has an activity *(e.g.,* to determine if the protein has nuclease activity that cleaves a target nucleic acid and/or some heterologous activity) can be any convenient assay *(e.g.,* any convenient nucleic acid cleavage assay that tests for nucleic acid cleavage). Suitable assays (*e.g.*, cleavage assays) will be known to one of ordinary skill in the art and can include adding a Cas9 guide RNA and a protein to a target nucleic acid.

In some cases, a chimeric Cas9 protein includes a heterologous polypeptide that has enzymatic activity that modifies target nucleic acid (*e.g.,* nuclease activity, methyltransferase activity, demethylase activity, DNA repair activity, DNA damage activity, deamination activity, dismutase activity, alkylation activity, depurination activity, oxidation activity, pyrimidine dimer forming activity, integrase activity, transposase activity, recombinase activity, polymerase activity, ligase activity, helicase activity, photolyase activity or glycosylase activity).

In other cases, a chimeric Cas9 protein includes a heterologous polypeptide that has enzymatic activity that modifies a polypeptide *(e.g.,* a histone) associated with target nucleic acid (*e.g*., methyltransferase activity, demethylase activity, acetyltransferase activity, deacetylase activity, kinase activity, phosphatase activity, ubiquitin ligase activity, deubiquitinating activity, adenylation activity, deadenylation activity, SUMOylating activity, deSUMOylating activity, ribosylation activity, deribosylation activity, myristoylation activity or demyristoylation activity).

Many Cas9 orthologs from a wide variety of species have been identified and in some cases the proteins share only a few identical amino acids. Identified Cas9 orthologs have similar domain architecture with a central HNH endonuclease domain and a split RuvC/RNaseH domain *(e.g.,* RuvCI, RuvCII, and RuvCIII) *(e.g.,* see Table 1). For example, a Cas9 protein can have 3 different regions (sometimes referred to as RuvC-I, RuvC-II, and RucC-III), that are not contiguous with respect to the primary amino acid sequence of the Cas9 protein, but fold together to form a RuvC domain once the protein is produced and folds. Thus, Cas9 proteins can be said to share at least 4 key motifs with a conserved architecture. Motifs 1, 2, and 4 are RuvC like motifs while motif 3 is an HNH-motif. The motifs set forth in Table 1 may not represent the entire RuvC-like and/or HNH domains as accepted in the art, but Table 1 does present motifs that can be used to help determine whether a given protein is a Cas9 protein.

**Table 1. Four motifs that are present in Cas9 sequences from various species.**

| The amino acids listed in this table are from the Cas9 from *S. pyogenes* (SEQ ID NO:5). | | | |
|---|---|---|---|
| **Motif #** | **Motif** | **Amino acids (residue #s)** | **Highly conserved** |
| 1 | RuvC-like I | IGLDIGTNSVGWAVI (7-21) (SEQ ID NO:1) | D10, G12, G17 |
| 2 | RuvC-like II | IVIEMARE (759-766) (SEQ ID NO:2) | E762 |
| 3 | HNH-motif | DVDHIVPQSFLKDDSIDNKVLTRSDK N (837-863) (SEQ ID NO:3) | H840, N854, N863 |
| 4 | RuvC-like III | HHAHDAYL (982-989) (SEQ ID NO:4) | H982, H983, A984, D986, A987 |

In some cases, a suitable Cas9 protein comprises an amino acid sequence having 4 motifs, each of motifs 1-4 having 60% or more, 70% or more, 75% or more, 80% or more, 85% or more, 90% or more, 95% or more, 99% or more or 100% amino acid sequence identity to motifs 1-4 as set forth in SEQ ID NOs: 1-4, respectively *(e.g.,* see Table 1), or to the corresponding portions in any of the amino acid sequences set forth in SEQ ID NOs: 5-816.

In other words, in some cases, a suitable Cas9 polypeptide comprises an amino acid sequence having 4 motifs, each of motifs 1-4 having 60% or more, 70% or more, 75% or more, 80% or more, 85% or more, 90% or more, 95% or more, 99% or more or 100% amino acid sequence identity to motifs 1-4 of the Cas9 amino acid sequence set forth in SEQ ID NO:5 *(e.g.,* the sequences set forth in SEQ ID NOs: 1-4, *e.g.,* see Table 1), or to the corresponding portions in any of the amino acid sequences set forth in SEQ ID NOs: 6-816.

In some cases, a suitable Cas9 protein comprises an amino acid sequence having 4 motifs, each of motifs 1-4 having 60% or more amino acid sequence identity to motifs 1-4 of the Cas9 amino acid sequence set forth as SEQ ID NO:5 (the motifs are in Table 1, and are set forth as SEQ ID NOs: 1-4, respectively), or to the corresponding portions in any of the amino acid sequences set forth in SEQ ID NOs: 6-816. In some cases, a suitable Cas9 protein comprises an amino acid sequence having 4 motifs, each of motifs 1-4 having 70% or more amino acid sequence identity to motifs 1-4 of the Cas9 amino acid sequence set forth as SEQ ID NO:5 (the motifs are in Table 1, and are set forth as SEQ ID NOs: 1-4, respectively), or to the corresponding portions in any of the amino acid sequences set forth in SEQ ID NOs: 6-816. In some cases, a suitable Cas9 protein comprises an amino acid sequence having 4 motifs, each of motifs 1-4 having 75% or more amino acid sequence identity to motifs 1-4 of the Cas9 amino acid sequence set forth as SEQ ID NO:5 (the motifs are in Table 1, and are set forth as SEQ ID NOs: 1-4, respectively), or to the corresponding portions in any of the amino acid sequences set forth in SEQ ID NOs: 6-816. In some cases, a suitable Cas9 protein comprises an amino acid sequence having 4 motifs, each of motifs 1-4 having 80% or more amino acid sequence identity to motifs 1-4 of the Cas9 amino acid sequence set forth as SEQ ID NO:5 (the motifs are in Table 1, and are set forth as SEQ ID NOs: 1-4, respectively), or to the corresponding portions in any of the amino acid sequences set forth in SEQ ID NOs: 6-816. In some cases, a suitable Cas9 protein comprises an amino acid sequence having 4 motifs, each of motifs 1-4 having 85% or more amino acid sequence identity to motifs 1-4 of the Cas9 amino acid sequence set forth as SEQ ID NO:5 (the motifs are in Table 1, and are set forth as SEQ ID NOs: 1-4, respectively), or to the corresponding portions in any of the amino acid sequences set forth in SEQ ID NOs: 6-816. In some cases, a suitable Cas9 protein comprises an amino acid sequence having 4 motifs, each of motifs 1-4 having 90% or more amino acid sequence identity to motifs 1-4 of the Cas9 amino acid sequence set forth as SEQ ID NO:5 (the motifs are in Table 1, and are set forth as SEQ ID NOs: 1-4, respectively), or to the corresponding portions in any of the amino acid sequences set forth in SEQ ID NOs: 6-816. In some cases, a suitable Cas9 protein comprises an amino acid sequence having 4 motifs, each of motifs 1-4 having 95% or more amino acid sequence identity to motifs 1-4 of the Cas9 amino acid sequence set forth as SEQ ID NO:5 (the motifs are in Table 1, and are set forth as SEQ ID NOs: 1-4, respectively), or to the corresponding portions in any of the amino acid sequences set forth in SEQ ID NOs: 6-816. In some cases, a suitable Cas9 protein comprises an amino acid sequence having 4 motifs, each of motifs 1-4 having 99% or more amino acid sequence identity to motifs 1-4 of the Cas9 amino acid sequence set forth as SEQ ID NO:5 (the motifs are in Table 1, and are set forth as SEQ ID NOs: 1-4, respectively), or to the corresponding portions in any of the amino acid sequences set forth in SEQ ID NOs: 6-816. In some cases, a suitable Cas9 protein comprises an amino acid sequence having 4 motifs, each of motifs 1-4 having 100% amino acid sequence identity to motifs 1-4 of the Cas9 amino acid sequence set forth as SEQ ID NO:5 (the motifs are in Table 1, and are set forth as SEQ ID NOs: 1-4, respectively), or to the corresponding portions in any of the amino acid sequences set forth in SEQ ID NOs: 6-816. Any Cas9 protein as defined above can be used as a Cas9 polypeptide, as part of a chimeric Cas9 polypeptide *(e.g.,* a Cas9 fusion protein), any of which can be used in an RNP of the present disclosure.

In some cases, a suitable Cas9 protein comprises an amino acid sequence having 60% or more, 70% or more, 75% or more, 80% or more, 85% or more, 90% or more, 95% or more, 99% or more or 100% amino acid sequence identity to amino acids 7-166 or 731-1003 of the Cas9 amino acid sequence set forth in SEQ ID NO:5, or to the corresponding portions in any of the amino acid sequences set forth as SEQ ID NOs: 6-816.

In some cases, a suitable Cas9 protein comprises an amino acid sequence having 60% or more amino acid sequence identity to amino acids 7-166 or 731-1003 of the Cas9 amino acid sequence set forth in SEQ ID NO:5, or to the corresponding portions in any of the amino acid sequences set forth as SEQ ID NOs: 6-816. In some cases, a suitable Cas9 protein comprises an amino acid sequence having 70% or more amino acid sequence identity to amino acids 7-166 or 731-1003 of the Cas9 amino acid sequence set forth in SEQ ID NO:5, or to the corresponding portions in any of the amino acid sequences set forth as SEQ ID NOs: 6-816. In some cases, a suitable Cas9 protein comprises an amino acid sequence having 75% or more amino acid sequence identity to amino acids 7-166 or 731-1003 of the Cas9 amino acid sequence set forth in SEQ ID NO:5, or to the corresponding portions in any of the amino acid sequences set forth as SEQ ID NOs: 6-816. In some cases, a suitable Cas9 protein comprises an amino acid sequence having 80% or more amino acid sequence identity to amino acids 7-166 or 731-1003 of the Cas9 amino acid sequence set forth in SEQ ID NO:5, or to the corresponding portions in any of the amino acid sequences set forth as SEQ ID NOs: 6-816. In some cases, a suitable Cas9 protein comprises an amino acid sequence having 85% or more amino acid sequence identity to amino acids 7-166 or 731-1003 of the Cas9 amino acid sequence set forth in SEQ ID NO:5, or to the corresponding portions in any of the amino acid sequences set forth as SEQ ID NOs: 6-816. In some cases, a suitable Cas9 protein comprises an amino acid sequence having 90% or more amino acid sequence identity to amino acids 7-166 or 731-1003 of the Cas9 amino acid sequence set forth in SEQ ID NO:5, or to the corresponding portions in any of the amino acid sequences set forth as SEQ ID NOs: 6-816. In some cases, a suitable Cas9 protein comprises an amino acid sequence having 95% or more amino acid sequence identity to amino acids 7-166 or 731-1003 of the Cas9 amino acid sequence set forth in SEQ ID NO:5, or to the corresponding portions in any of the amino acid sequences set forth as SEQ ID NOs: 6-816. In some cases, a suitable Cas9 protein comprises an amino acid sequence having 99% or more amino acid sequence identity to amino acids 7-166 or 731-1003 of the Cas9 amino acid sequence set forth in SEQ ID NO:5, or to the corresponding portions in any of the amino acid sequences set forth as SEQ ID NOs: 6-816. In some cases, a suitable Cas9 protein comprises an amino acid sequence having 100% amino acid sequence identity to amino acids 7-166 or 731-1003 of the Cas9 amino acid sequence set forth in SEQ ID NO:5, or to the corresponding portions in any of the amino acid sequences set forth as SEQ ID NOs: 6-816. Any Cas9 protein as defined above can be used as a Cas9 polypeptide, as part of a chimeric Cas9 polypeptide *(e.g.,* a Cas9 fusion protein), any of which can be used in an RNP of the present disclosure

In some cases, a suitable Cas9 protein comprises an amino acid sequence having 60% or more, 70% or more, 75% or more, 80% or more, 85% or more, 90% or more, 95% or more, 99% or more or 100% amino acid sequence identity to the Cas9 amino acid sequence set forth in SEQ ID NO:5, or to any of the amino acid sequences set forth as SEQ ID NOs: 6-816.

In some cases, a suitable Cas9 protein comprises an amino acid sequence having 60% or more amino acid sequence identity to the Cas9 amino acid sequence set forth in SEQ ID NO:5, or to any of the amino acid sequences set forth as SEQ ID NOs: 6-816. In some cases, a suitable Cas9 protein comprises an amino acid sequence having 70% or more amino acid sequence identity to the Cas9 amino acid sequence set forth in SEQ ID NO:5, or to any of the amino acid sequences set forth as SEQ ID NOs: 6-816. In some cases, a suitable Cas9 protein comprises an amino acid sequence having 75% or more amino acid sequence identity to the Cas9 amino acid sequence set forth in SEQ ID NO:5, or to any of the amino acid sequences set forth as SEQ ID NOs: 6-816. In some cases, a suitable Cas9 protein comprises an amino acid sequence having 80% or more amino acid sequence identity to the Cas9 amino acid sequence set forth in SEQ ID NO:5, or to any of the amino acid sequences set forth as SEQ ID NOs: 6-816. In some cases, a suitable Cas9 protein comprises an amino acid sequence having 85% or more amino acid sequence identity to the Cas9 amino acid sequence set forth in SEQ ID NO:5, or to any of the amino acid sequences set forth as SEQ ID NOs: 6-816. In some cases, a suitable Cas9 protein comprises an amino acid sequence having 90% or more amino acid sequence identity to the Cas9 amino acid sequence set forth in SEQ ID NO:5, or to any of the amino acid sequences set forth as SEQ ID NOs: 6-816. In some cases, a suitable Cas9 protein comprises an amino acid sequence having 95% or more amino acid sequence identity to the Cas9 amino acid sequence set forth in SEQ ID NO:5, or to any of the amino acid sequences set forth as SEQ ID NOs: 6-816. In some cases, a suitable Cas9 protein comprises an amino acid sequence having 99% or more amino acid sequence identity to the Cas9 amino acid sequence set forth in SEQ ID NO:5, or to any of the amino acid sequences set forth as SEQ ID NOs: 6-816. In some cases, a suitable Cas9 protein comprises an amino acid sequence having 100% amino acid sequence identity to the Cas9 amino acid sequence set forth in SEQ ID NO:5, or to any of the amino acid sequences set forth as SEQ ID NOs: 6-816. Any Cas9 protein as defined above can be used as a Cas9 polypeptide, as part of a chimeric Cas9 polypeptide *(e.g.,* a Cas9 fusion protein), any of which can be used in an RNP of the present disclosure.

In some cases, a Cas9 protein comprises 4 motifs (as listed in Table 1), at least one with (or each with) amino acid sequences having 75% or more, 80% or more, 85% or more, 90% or more, 95% or more, 99% or more or 100% amino acid sequence identity to each of the 4 motifs listed in Table 1 (SEQ ID NOs:1-4), or to the corresponding portions in any of the amino acid sequences set forth as SEQ ID NOs: 6-816.

As used herein, the term "Cas9 protein" encompasses a "chimeric Cas9 protein." As used herein, the term "Cas9 protein" encompasses a variant Cas9 that is a nickase. As used herein, the term "Cas9 protein" encompasses a variant Cas9 that exhibits reduced enzymatic activity *(e.g.,* a "dead Cas9" or "dCas9").

### Variant Cas9 proteins - nickases and dCas9

In some cases, a Cas9 protein is a variant Cas9 protein. A variant Cas9 protein has an amino acid sequence that is different by at least one amino acid *(e.g.,* has a deletion, insertion, substitution, fusion) when compared to the amino acid sequence of a corresponding wild type Cas9 protein. In some instances, the variant Cas9 protein has an amino acid change *(e.g.,* deletion, insertion, or substitution) that reduces the nuclease activity of the Cas9 protein. For example, in some instances, the variant Cas9 protein has 50% or less, 40% or less, 30% or less, 20% or less, 10% or less, 5% or less, or 1% or less of the nuclease activity of the corresponding wild-type Cas9 protein. In some cases, the variant Cas9 protein has no substantial nuclease activity. When a Cas9 protein is a variant Cas9 protein that has no substantial nuclease activity, it can be referred to as "dCas9." A protein *(e.g.,* a class 2 CRISPR/Cas protein, *e.g.,* a Cas9 protein) that cleaves one strand but not the other of a double stranded target nucleic acid is referred to herein as a "nickase" *(e.g.,* a "nickase Cas9").

In some cases, a variant Cas9 protein can cleave the complementary strand (sometimes referred to in the art as the target strand) of a target nucleic acid but has reduced ability to cleave the non-complementary strand (sometimes referred to in the art as the non-target strand) of a target nucleic acid. For example, the variant Cas9 protein can have a mutation (amino acid substitution) that reduces the function of the RuvC domain. Thus, the Cas9 protein can be a nickase that cleaves the complementary strand, but does not cleave the non-complementary strand. As a non-limiting example, in some embodiments, a variant Cas9 protein has a mutation at an amino acid position corresponding to residue D10 *(e.g.,* D10A, aspartate to alanine) of SEQ ID NO:5 (or the corresponding position of any of the proteins set forth in SEQ ID NOs: 6-261 and 265-816) and can therefore cleave the complementary strand of a double stranded target nucleic acid but has reduced ability to cleave the non-complementary strand of a double stranded target nucleic acid (thus resulting in a single strand break (SSB) instead of a double strand break (DSB) when the variant Cas9 protein cleaves a double stranded target nucleic acid) (see, for example, Jinek et al., Science. 2012 Aug 17;337(6096):816-21). See, *e.g*., SEQ ID NO:262

In some cases, a variant Cas9 protein can cleave the non-complementary strand of a target nucleic acid but has reduced ability to cleave the complementary strand of the target nucleic acid. For example, the variant Cas9 protein can have a mutation (amino acid substitution) that reduces the function of the HNH domain. Thus, the Cas9 protein can be a nickase that cleaves the non-complementary strand, but does not cleave the complementary strand. As a non-limiting example, in some embodiments, the variant Cas9 protein has a mutation at an amino acid position corresponding to residue H840 *(e.g.,* an H840A mutation, histidine to alanine) of SEQ ID NO:5 (or the corresponding position of any of the proteins set forth as SEQ ID NOs: 6-261and 265-816) and can therefore cleave the non-complementary strand of the target nucleic acid but has reduced ability to cleave *(e.g.,* does not cleave) the complementary strand of the target nucleic acid. Such a Cas9 protein has a reduced ability to cleave a target nucleic acid *(e.g.,* a single stranded target nucleic acid) but retains the ability to bind a target nucleic acid *(e.g.,* a single stranded target nucleic acid). See, *e.g.,* SEQ ID NO:263

In some cases, a variant Cas9 protein has a reduced ability to cleave both the complementary and the non-complementary strands of a double stranded target nucleic acid. As a non-limiting example, in some cases, the variant Cas9 protein harbors mutations at amino acid positions corresponding to residues D10 and H840 *(e.g.,* D10A and H840A) of SEQ ID NO:5 (or the corresponding residues of any of the proteins set forth as SEQ ID NOs: 6-261 and 265-816) such that the polypeptide has a reduced ability to cleave *(e.g.,* does not cleave) both the complementary and the non-complementary strands of a target nucleic acid. Such a Cas9 protein has a reduced ability to cleave a target nucleic acid *(e.g.,* a single stranded or double stranded target nucleic acid) but retains the ability to bind a target nucleic acid. A Cas9 protein that cannot cleave target nucleic acid *(e.g.,* due to one or more mutations, *e.g*., in the catalytic domains of the RuvC and HNH domains) is referred to as a "dead" Cas9 or simply "dCas9." See, *e.g.,* SEQ ID NO:264

Other residues can be mutated to achieve the above effects (i.e. inactivate one or the other nuclease portions). As non-limiting examples, residues D10, G12, G17, E762, H840, N854, N863, H982, H983, A984, D986, and/or A987 of SEQ ID NO:5 (or the corresponding mutations of any of the proteins set forth as SEQ ID NOs: 6-816) can be altered *(i.e.,* substituted). Also, mutations other than alanine substitutions are suitable.

In some embodiments, a variant Cas9 protein that has reduced catalytic activity (*e.g.*, when a Cas9 protein has a D10, G12, G17, E762, H840, N854, N863, H982, H983, A984, D986, and/or a A987 mutation of SEQ ID NO:5 or the corresponding mutations of any of the proteins set forth as SEQ ID NOs: 6-816, *e.g.,* D10A, G12A, G17A, E762A, H840A, N854A, N863A, H982A, H983A, A984A, and/or D986A), the variant Cas9 protein can still bind to target nucleic acid in a site-specific manner (because it is still guided to a target nucleic acid sequence by a Cas9 guide RNA) as long as it retains the ability to interact with the Cas9 guide RNA

In addition to the above, a variant Cas9 protein can have the same parameters for sequence identity as described above for Cas9 proteins. Thus, in some cases, a suitable variant Cas9 protein comprises an amino acid sequence having 4 motifs, each of motifs 1-4 having 60% or more, 70% or more, 75% or more, 80% or more, 85% or more, 90% or more, 95% or more, 99% or more or 100% amino acid sequence identity to motifs 1-4 of the Cas9 amino acid sequence set forth as SEQ ID NO:5 (the motifs are in Table 1, below, and are set forth as SEQ ID NOs: 1-4, respectively), or to the corresponding portions in any of the amino acid sequences set forth in SEQ ID NOs: 6-816.

In some cases, a suitable variant Cas9 protein comprises an amino acid sequence having 4 motifs, each of motifs 1-4 having 60% or more amino acid sequence identity to motifs 1-4 of the Cas9 amino acid sequence set forth as SEQ ID NO:5 (the motifs are in Table 1, below, and are set forth as SEQ ID NOs: 1-4, respectively), or to the corresponding portions in any of the amino acid sequences set forth in SEQ ID NOs: 6-816. In some cases, a suitable variant Cas9 protein comprises an amino acid sequence having 4 motifs, each of motifs 1-4 having 70% or more amino acid sequence identity to motifs 1-4 of the Cas9 amino acid sequence set forth as SEQ ID NO:5 (the motifs are in Table 1, below, and are set forth as SEQ ID NOs: 1-4, respectively), or to the corresponding portions in any of the amino acid sequences set forth in SEQ ID NOs: 6-816. In some cases, a suitable variant Cas9 protein comprises an amino acid sequence having 4 motifs, each of motifs 1-4 having 75% or more amino acid sequence identity to motifs 1-4 of the Cas9 amino acid sequence set forth as SEQ ID NO:5 (the motifs are in Table 1, below, and are set forth as SEQ ID NOs: 1-4, respectively), or to the corresponding portions in any of the amino acid sequences set forth in SEQ ID NOs: 6-816. In some cases, a suitable variant Cas9 protein comprises an amino acid sequence having 4 motifs, each of motifs 1-4 having 80% or more amino acid sequence identity to motifs 1-4 of the Cas9 amino acid sequence set forth as SEQ ID NO:5 (the motifs are in Table 1, below, and are set forth as SEQ ID NOs: 1-4, respectively), or to the corresponding portions in any of the amino acid sequences set forth in SEQ ID NOs: 6-816. In some cases, a suitable variant Cas9 protein comprises an amino acid sequence having 4 motifs, each of motifs 1-4 having 85% or more amino acid sequence identity to motifs 1-4 of the Cas9 amino acid sequence set forth as SEQ ID NO:5 (the motifs are in Table 1, below, and are set forth as SEQ ID NOs: 1-4, respectively), or to the corresponding portions in any of the amino acid sequences set forth in SEQ ID NOs: 6-816. In some cases, a suitable variant Cas9 protein comprises an amino acid sequence having 4 motifs, each of motifs 1-4 having 90% or more amino acid sequence identity to motifs 1-4 of the Cas9 amino acid sequence set forth as SEQ ID NO:5 (the motifs are in Table 1, below, and are set forth as SEQ ID NOs: 1-4, respectively), or to the corresponding portions in any of the amino acid sequences set forth in SEQ ID NOs: 6-816. In some cases, a suitable variant Cas9 protein comprises an amino acid sequence having 4 motifs, each of motifs 1-4 having 95% or more amino acid sequence identity to motifs 1-4 of the Cas9 amino acid sequence set forth as SEQ ID NO:5 (the motifs are in Table 1, below, and are set forth as SEQ ID NOs: 1-4, respectively), or to the corresponding portions in any of the amino acid sequences set forth in SEQ ID NOs: 6-816. In some cases, a suitable variant Cas9 protein comprises an amino acid sequence having 4 motifs, each of motifs 1-4 having 99% or more amino acid sequence identity to motifs 1-4 of the Cas9 amino acid sequence set forth as SEQ ID NO:5 (the motifs are in Table 1, below, and are set forth as SEQ ID NOs: 1-4, respectively), or to the corresponding portions in any of the amino acid sequences set forth in SEQ ID NOs: 6-816. In some cases, a suitable variant Cas9 protein comprises an amino acid sequence having 4 motifs, each of motifs 1-4 having 100% amino acid sequence identity to motifs 1-4 of the Cas9 amino acid sequence set forth as SEQ ID NO:5 (the motifs are in Table 1, below, and are set forth as SEQ ID NOs: 1-4, respectively), or to the corresponding portions in any of the amino acid sequences set forth in SEQ ID NOs: 6-816

In some cases, a suitable variant Cas9 protein comprises an amino acid sequence having 60% or more, 70% or more, 75% or more, 80% or more, 85% or more, 90% or more, 95% or more, 99% or more, or 100% amino acid sequence identity to amino acids 7-166 or 731-1003 of the Cas9 amino acid sequence set forth in SEQ ID NO:5, or to the corresponding portions in any of the amino acid sequences set forth as SEQ ID NOs: 6-816

In some cases, a suitable variant Cas9 protein comprises an amino acid sequence having 60% or more amino acid sequence identity to amino acids 7-166 or 731-1003 of the Cas9 amino acid sequence set forth in SEQ ID NO:5, or to the corresponding portions in any of the amino acid sequences set forth as SEQ ID NOs: 6-816. In some cases, a suitable variant Cas9 protein comprises an amino acid sequence having 70% or more amino acid sequence identity to amino acids 7-166 or 731-1003 of the Cas9 amino acid sequence set forth in SEQ ID NO:5, or to the corresponding portions in any of the amino acid sequences set forth as SEQ ID NOs: 6-816. In some cases, a suitable variant Cas9 protein comprises an amino acid sequence having 75% or more amino acid sequence identity to amino acids 7-166 or 731-1003 of the Cas9 amino acid sequence set forth in SEQ ID NO:5, or to the corresponding portions in any of the amino acid sequences set forth as SEQ ID NOs: 6-816. In some cases, a suitable variant Cas9 protein comprises an amino acid sequence having 80% or more amino acid sequence identity to amino acids 7-166 or 731-1003 of the Cas9 amino acid sequence set forth in SEQ ID NO:5, or to the corresponding portions in any of the amino acid sequences set forth as SEQ ID NOs: 6-816. In some cases, a suitable variant Cas9 protein comprises an amino acid sequence having 85% or more amino acid sequence identity to amino acids 7-166 or 731-1003 of the Cas9 amino acid sequence set forth in SEQ ID NO:5, or to the corresponding portions in any of the amino acid sequences set forth as SEQ ID NOs: 6-816. In some cases, a suitable variant Cas9 protein comprises an amino acid sequence having 90% or more amino acid sequence identity to amino acids 7-166 or 731-1003 of the Cas9 amino acid sequence set forth in SEQ ID NO:5, or to the corresponding portions in any of the amino acid sequences set forth as SEQ ID NOs: 6-816. In some cases, a suitable variant Cas9 protein comprises an amino acid sequence having 95% or more amino acid sequence identity to amino acids 7-166 or 731-1003 of the Cas9 amino acid sequence set forth in SEQ ID NO:5, or to the corresponding portions in any of the amino acid sequences set forth as SEQ ID NOs: 6-816. In some cases, a suitable variant Cas9 protein comprises an amino acid sequence having 99% or more amino acid sequence identity to amino acids 7-166 or 731-1003 of the Cas9 amino acid sequence set forth in SEQ ID NO:5, or to the corresponding portions in any of the amino acid sequences set forth as SEQ ID NOs: 6-816. In some cases, a suitable variant Cas9 protein comprises an amino acid sequence having 100% amino acid sequence identity to amino acids 7-166 or 731-1003 of the Cas9 amino acid sequence set forth in SEQ ID NO:5, or to the corresponding portions in any of the amino acid sequences set forth as SEQ ID NOs: 6-816.

In some cases, a suitable variant Cas9 protein comprises an amino acid sequence having 60% or more, 70% or more, 75% or more, 80% or more, 85% or more, 90% or more, 95% or more, 99% or more, or 100% amino acid sequence identity to the Cas9 amino acid sequence set forth in SEQ ID NO:5, or to any of the amino acid sequences set forth as SEQ ID NOs: 6-816. In some cases, a suitable variant Cas9 protein comprises an amino acid sequence having 60% or more amino acid sequence identity to the Cas9 amino acid sequence set forth in SEQ ID NO:5, or to any of the amino acid sequences set forth as SEQ ID NOs: 6-816. In some cases, a suitable variant Cas9 protein comprises an amino acid sequence having 70% or more amino acid sequence identity to the Cas9 amino acid sequence set forth in SEQ ID NO:5, or to any of the amino acid sequences set forth as SEQ ID NOs: 6-816. In some cases, a suitable variant Cas9 protein comprises an amino acid sequence having 75% or more amino acid sequence identity to the Cas9 amino acid sequence set forth in SEQ ID NO:5, or to any of the amino acid sequences set forth as SEQ ID NOs: 6-816. In some cases, a suitable variant Cas9 protein comprises an amino acid sequence having 80% or more amino acid sequence identity to the Cas9 amino acid sequence set forth in SEQ ID NO:5, or to any of the amino acid sequences set forth as SEQ ID NOs: 6-816. In some cases, a suitable variant Cas9 protein comprises an amino acid sequence having 85% or more amino acid sequence identity to the Cas9 amino acid sequence set forth in SEQ ID NO:5, or to any of the amino acid sequences set forth as SEQ ID NOs: 6-816. In some cases, a suitable variant Cas9 protein comprises an amino acid sequence having 90% or more amino acid sequence identity to the Cas9 amino acid sequence set forth in SEQ ID NO:5, or to any of the amino acid sequences set forth as SEQ ID NOs: 6-816. In some cases, a suitable variant Cas9 protein comprises an amino acid sequence having 95% or more amino acid sequence identity to the Cas9 amino acid sequence set forth in SEQ ID NO:5, or to any of the amino acid sequences set forth as SEQ ID NOs: 6-816. In some cases, a suitable variant Cas9 protein comprises an amino acid sequence having 99% or more amino acid sequence identity to the Cas9 amino acid sequence set forth in SEQ ID NO:5, or to any of the amino acid sequences set forth as SEQ ID NOs: 6-816. In some cases, a suitable variant Cas9 protein comprises an amino acid sequence having 100% amino acid sequence identity to the Cas9 amino acid sequence set forth in SEQ ID NO:5, or to any of the amino acid sequences set forth as SEQ ID NOs: 6-816.

### Type V and Type VI CRISPR/Cas Endonucleases

In some cases, a genome editing composition of the present disclosure includes a type V or type VI CRISPR/Cas endonuclease (*i.e.,* the genome editing endonuclease is a type V or type VI CRISPR/Cas endonuclease) *(e.g.,* Cpfl, C2c1, C2c2, C2c3). Type V and type VI CRISPR/Cas endonucleases are a type of class 2 CRISPR/Cas endonuclease. Examples of type V CRISPR/Cas endonucleases include but are not limited to: Cpfl, C2c1, and C2c3. An example of a type VI CRISPR/Cas endonuclease is C2c2. In some cases, a subject genome editing composition includes a type V CRISPR/Cas endonuclease *(e.g.,* Cpfl, C2c1, C2c3). In some cases, a Type V CRISPR/Cas endonuclease is a Cpfl protein. In some cases, a subject genome editing composition includes a type VI CRISPR/Cas endonuclease (*e.g*., C2c2)

Like type II CRISPR/Cas endonucleases, type V and VI CRISPR/Cas endonucleases form a complex with a corresponding guide RNA. The guide RNA provides target specificity to an endonuclease-guide RNA RNP complex by having a nucleotide sequence (a guide sequence) that is complementary to a sequence (the target site) of a target nucleic acid (as described elsewhere herein). The endonuclease of the complex provides the site-specific activity. In other words, the endonuclease is guided to a target site *(e.g.,* stabilized at a target site) within a target nucleic acid sequence *(e.g.,* a chromosomal sequence or an extrachromosomal sequence, *e.g.,* an episomal sequence, a minicircle sequence, a mitochondrial sequence, a chloroplast sequence, *etc.)* by virtue of its association with the protein-binding segment of the guide RNA.

Examples and guidance related to type V and type VI CRISPR/Cas proteins *(e.g.,* cpf1, C2c1, C2c2, and C2c3 guide RNAs) can be found in the art, for example, see Zetsche et al, Cell. 2015 Oct 22;163(3):759-71; Makarova et al, Nat Rev Microbiol. 2015 Nov;13(11):722-36; and Shmakov et al., Mol Cell. 2015 Nov 5;60(3):385-97.

In some cases, the Type V or type VI CRISPR/Cas endonuclease *(e.g.,* Cpfl, C2c1, C2c2, C2c3) is enzymatically active, *e.g.,* the Type V or type VI CRISPR/Cas polypeptide, when bound to a guide RNA, cleaves a target nucleic acid. In some cases, the Type V or type VI CRISPR/Cas endonuclease *(e.g.,* Cpfl, C2c1, C2c2, C2c3) exhibits reduced enzymatic activity relative to a corresponding wild-type a Type V or type VI CRISPR/Cas endonuclease *(e.g.,* Cpfl, C2c1, C2c2, C2c3), and retains DNA binding activity.

In some cases a type V CRISPR/Cas endonuclease is a Cpfl protein. In some cases, a Cpfl protein comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to the Cpfl amino acid sequence set forth in any of SEQ ID NOs: 1088-1092. In some cases, a Cpfl protein comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to a contiguous stretch of from 100 amino acids to 200 amino acids (aa), from 200 aa to 400 aa, from 400 aa to 600 aa, from 600 aa to 800 aa, from 800 aa to 1000 aa, from 1000 aa to 1100 aa, from 1100 aa to 1200 aa, or from 1200 aa to 1300 aa, of the Cpfl amino acid sequence set forth in any of SEQ ID NOs: 1088-1092

In some cases, a Cpfl protein comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to the RuvCI domain of the Cpfl amino acid sequence set forth in any of SEQ ID NOs: 1088-1092. In some cases, a Cpfl protein comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to the RuvCII domain of the Cpfl amino acid sequence set forth in any of SEQ ID NOs: 1088-1092. In some cases, a Cpfl protein comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to the RuvCIII domain of the Cpfl amino acid sequence set forth in any of SEQ ID NOs: 1088-1092. In some cases, a Cpfl protein comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to the RuvCI, RuvCII, and RuvCIII domains of the Cpfl amino acid sequence set forth in any of SEQ ID NOs: 1088-1092

In some cases, the Cpfl protein exhibits reduced enzymatic activity relative to a wild-type Cpfl protein *(e.g.,* relative to a Cpfl protein comprising the amino acid sequence set forth in any of SEQ ID NOs: 1088-1092), and retains DNA binding activity. In some cases, a Cpfl protein comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to the Cpfl amino acid sequence set forth in any of SEQ ID NOs: 1088-1092; and comprises an amino acid substitution (*e.g.*, a D→A substitution) at an amino acid residue corresponding to amino acid 917 of the Cpfl amino acid sequence set forth in SEQ ID NO:1088. In some cases, a Cpfl protein comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to the Cpfl amino acid sequence set forth in any of SEQ ID NOs: 1088-1092; and comprises an amino acid substitution (*e.g.*, an E→A substitution) at an amino acid residue corresponding to amino acid 1006 of the Cpfl amino acid sequence set forth in SEQ ID NO:1088. In some cases, a Cpfl protein comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to the Cpfl amino acid sequence set forth in any of SEQ ID NOs: 1088-1092; and comprises an amino acid substitution (*e.g.*, a D→A substitution) at an amino acid residue corresponding to amino acid 1255 of the Cpfl amino acid sequence set forth in SEQ ID NO:1088

In some cases, a suitable Cpfl protein comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to the Cpfl amino acid sequence set forth in any of SEQ ID NOs: 1088-1092

In some cases a type V CRISPR/Cas endonuclease is a C2c1 protein (examples include those set forth as SEQ ID NOs: 1112-1119). In some cases, a C2c1 protein comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to the C2c1 amino acid sequence set forth in any of SEQ ID NOs: 1112-1119. In some cases, a C2c1 protein comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to a contiguous stretch of from 100 amino acids to 200 amino acids (aa), from 200 aa to 400 aa, from 400 aa to 600 aa, from 600 aa to 800 aa, from 800 aa to 1000 aa, from 1000 aa to 1100 aa, from 1100 aa to 1200 aa, or from 1200 aa to 1300 aa, of the C2c1 amino acid sequence set forth in any of SEQ ID NOs: 1112-1119

In some cases, a C2c1 protein comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to the RuvCI domain of the C2c1 amino acid sequences set forth in any of SEQ ID NOs: 1112-1119). In some cases, a C2c1 protein comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to the RuvCII domain of the C2c1 amino acid sequence set forth in any of SEQ ID NOs: 1112-1119. In some cases, a C2c1 protein comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to the RuvCIII domain of the C2c1 amino acid sequence set forth in any of SEQ ID NOs: 1112-1119. In some cases, a C2c1 protein comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to the RuvCI, RuvCII, and RuvCIII domains of the C2c1 amino acid sequence set forth in any of SEQ ID NOs: 1112-1119

In some cases, the C2c1 protein exhibits reduced enzymatic activity relative to a wild-type C2c1 protein (*e.g*., relative to a C2c1 protein comprising the amino acid sequence set forth in any of SEQ ID NOs: 1112-1119), and retains DNA binding activity. In some cases, a suitable C2c1 protein comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to the C2c1 amino acid sequence set forth in any of SEQ ID NOs: 1112-1119.

In some cases a type V CRISPR/Cas endonuclease is a C2c3 protein (examples include those set forth as SEQ ID NOs: 1120-1123). In some cases, a C2c3 protein comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to the C2c3 amino acid sequence set forth in any of SEQ ID NOs: 1120-1123. In some cases, a C2c3 protein comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to a contiguous stretch of from 100 amino acids to 200 amino acids (aa), from 200 aa to 400 aa, from 400 aa to 600 aa, from 600 aa to 800 aa, from 800 aa to 1000 aa, from 1000 aa to 1100 aa, from 1100 aa to 1200 aa, or from 1200 aa to 1300 aa, of the C2c3 amino acid sequence set forth in any of SEQ ID NOs: 1120-1123

In some cases, a C2c3 protein comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to the RuvCI domain of the C2c3 amino acid sequence set forth in any of SEQ ID NOs: 1120-1123. In some cases, a C2c3 protein comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to the RuvCII domain of the C2c3 amino acid sequence set forth in any of SEQ ID NOs: 1120-1123. In some cases, a C2c3 protein comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to the RuvCIII domain of the C2c3 amino acid sequence set forth in any of SEQ ID NOs: 1120-1123. In some cases, a C2c3 protein comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to the RuvCI, RuvCII, and RuvCIII domains of the C2c3 amino acid sequence set forth in any of SEQ ID NOs: 1120-1123.

In some cases, the C2c3 protein exhibits reduced enzymatic activity relative to a wild-type C2c3 protein (*e.g.*, relative to a C2c3 protein comprising the amino acid sequence set forth in any of SEQ ID NOs: 1120-1123), and retains DNA binding activity. In some cases, a suitable C2c3 protein comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to the C2c3 amino acid sequence set forth in any of SEQ ID NOs: 1120-1123

In some cases a type VI CRISPR/Cas endonuclease is a C2c2 protein (examples include those set forth as SEQ ID NOs: 1124-1135). In some cases, a C2c2 protein comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to the C2c2 amino acid sequence set forth in any of SEQ ID NOs: 1124-1135. In some cases, a C2c2 protein comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to a contiguous stretch of from 100 amino acids to 200 amino acids (aa), from 200 aa to 400 aa, from 400 aa to 600 aa, from 600 aa to 800 aa, from 800 aa to 1000 aa, from 1000 aa to 1100 aa, from 1100 aa to 1200 aa, or from 1200 aa to 1300 aa, of the C2c2 amino acid sequence set forth in any of SEQ ID NOs: 1124-1135.

In some cases, a C2c2 protein comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to the RuvCI domain of the C2c2 amino acid sequence set forth in any of SEQ ID NOs: 1124-1135. In some cases, a C2c2 protein comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to the RuvCII domain of the C2c2 amino acid sequence set forth in any of SEQ ID NOs: 1124-1135. In some cases, a C2c2 protein comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to the RuvCIII domain of the C2c2 amino acid sequence set forth in any of SEQ ID NOs: 1124-1135. In some cases, a C2c2 protein comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to the RuvCI, RuvCII, and RuvCIII domains of the C2c2 amino acid sequence set forth in any of SEQ ID NOs: 1124-1135.

In some cases, the C2c2 protein exhibits reduced enzymatic activity relative to a wild-type C2c2 protein (*e.g.*, relative to a C2c2 protein comprising the amino acid sequence set forth in any of SEQ ID NOs: 1124-1135), and retains DNA binding activity. In some cases, a suitable C2c2 protein comprises an amino acid sequence having at least 30%, at least 35%, at least 40%, at least 45%, at least 50%, at least 55%, at least 60%, at least 65%, at least 70%, at least 75%, at least 80%, at least 85%, at least 90%, at least 95%, at least 90%, or 100%, amino acid sequence identity to the C2c2 amino acid sequence set forth in any of SEQ ID NOs: 1124-1135.

### Guide RNA (for CRISPR/Cas endonucleases)

A nucleic acid molecule that binds to a class 2 CRISPR/Cas endonuclease *(e.g.,* a Cas9 protein; a type V or type VI CRISPR/Cas protein; a Cpfl protein; *etc.)* and targets the complex to a specific location within a target nucleic acid is referred to herein as a "guide RNA" or "CRISPR/Cas guide nucleic acid" or "CRISPR/Cas guide RNA."

A guide RNA provides target specificity to the complex (the RNP complex) by including a targeting segment, which includes a guide sequence (also referred to herein as a targeting sequence), which is a nucleotide sequence that is complementary to a sequence of a target nucleic acid

A guide RNA can be referred to by the protein to which it corresponds. For example, when the class 2 CRISPR/Cas endonuclease is a Cas9 protein, the corresponding guide RNA can be referred to as a "Cas9 guide RNA." Likewise, as another example, when the class 2 CRISPR/Cas endonuclease is a Cpfl protein, the corresponding guide RNA can be referred to as a "Cpfl guide RNA."

In some embodiments, a guide RNA includes two separate nucleic acid molecules: an "activator" and a "targeter" and is referred to herein as a "dual guide RNA", a "double-molecule guide RNA", a "two-molecule guide RNA", or a "dgRNA." In some embodiments, the guide RNA is one molecule *(e.g.,* for some class 2 CRISPR/Cas proteins, the corresponding guide RNA is a single molecule; and in some cases, an activator and targeter are covalently linked to one another, *e.g.,* via intervening nucleotides), and the guide RNA is referred to as a "single guide RNA", a "single-molecule guide RNA," a "one-molecule guide RNA", or simply "sgRNA."

### Cas9 Guide RNA

A nucleic acid molecule that binds to a Cas9 protein and targets the complex to a specific location within a target nucleic acid is referred to herein as a "Cas9 guide RNA."

A Cas9 guide RNA (can be said to include two segments, a first segment (referred to herein as a "targeting segment"); and a second segment (referred to herein as a "protein-binding segment"). By "segment" it is meant a segment/section/region of a molecule, *e.g.,* a contiguous stretch of nucleotides in a nucleic acid molecule. A segment can also mean a region/section of a complex such that a segment may comprise regions of more than one molecule.

The first segment (targeting segment) of a Cas9 guide RNA includes a nucleotide sequence (a guide sequence) that is complementary to (and therefore hybridizes with) a specific sequence (a target site) within a target nucleic acid *(e.g.,* a target ssRNA, a target ssDNA, the complementary strand of a double stranded target DNA, *etc.).* The protein-binding segment (or "protein-binding sequence") interacts with (binds to) a Cas9 polypeptide. The protein-binding segment of a subject Cas9 guide RNA includes two complementary stretches of nucleotides that hybridize to one another to form a double stranded RNA duplex (dsRNA duplex). Site-specific binding and/or cleavage of a target nucleic acid *(e.g.,* genomic DNA) can occur at locations *(e.g.,* target sequence of a target locus) determined by base-pairing complementarity between the Cas9 guide RNA (the guide sequence of the Cas9 guide RNA) and the target nucleic acid

A Cas9 guide RNA and a Cas9 protein form a complex *(e.g.,* bind via noncovalent interactions). The Cas9 guide RNA provides target specificity to the complex by including a targeting segment, which includes a guide sequence (a nucleotide sequence that is complementary to a sequence of a target nucleic acid). The Cas9 protein of the complex provides the site-specific activity (*e.g.*, cleavage activity or an activity provided by the Cas9 protein when the Cas9 protein is a Cas9 fusion polypeptide, *i.e.,* has a fusion partner). In other words, the Cas9 protein is guided to a target nucleic acid sequence *(e.g.,* a target sequence in a chromosomal nucleic acid, *e.g.,* a chromosome; a target sequence in an extrachromosomal nucleic acid, *e.g.,* an episomal nucleic acid, a minicircle, an ssRNA, an ssDNA, *etc*.; a target sequence in a mitochondrial nucleic acid; a target sequence in a chloroplast nucleic acid; a target sequence in a plasmid; a target sequence in a viral nucleic acid; *etc.)* by virtue of its association with the Cas9 guide RNA.

The "guide sequence" also referred to as the "targeting sequence" of a Cas9 guide RNA can be modified so that the Cas9 guide RNA can target a Cas9 protein to any desired sequence of any desired target nucleic acid, with the exception that the protospacer adjacent motif (PAM) sequence can be taken into account. Thus, for example, a Cas9 guide RNA can have a targeting segment with a sequence (a guide sequence) that has complementarity with *(e.g.,* can hybridize to) a sequence in a nucleic acid in a eukaryotic cell, *e.g.,* a viral nucleic acid, a eukaryotic nucleic acid *(e.g.,* a eukaryotic chromosome, chromosomal sequence, a eukaryotic RNA, *etc.),* and the like.

In some embodiments, a Cas9 guide RNA includes two separate nucleic acid molecules: an "activator" and a "targeter" and is referred to herein as a "dual Cas9 guide RNA", a "double-molecule Cas9 guide RNA", or a "two-molecule Cas9 guide RNA" a "dual guide RNA", or a "dgRNA." In some embodiments, the activator and targeter are covalently linked to one another *(e.g.,* via intervening nucleotides) and the guide RNA is referred to as a "single guide RNA", a "Cas9 single guide RNA", a "single-molecule Cas9 guide RNA," or a "one-molecule Cas9 guide RNA", or simply "sgRNA."

A Cas9 guide RNA comprises a crRNA-like ("CRISPR RNA" / "targeter" / "crRNA" / "crRNA repeat") molecule and a corresponding tracrRNA-like ("trans-acting CRISPR RNA" / "activator" / "tracrRNA") molecule. A crRNA-like molecule (targeter) comprises both the targeting segment (single stranded) of the Cas9 guide RNA and a stretch ("duplex-forming segment") of nucleotides that forms one half of the dsRNA duplex of the protein-binding segment of the Cas9 guide RNA. A corresponding tracrRNA-like molecule (activator / tracrRNA) comprises a stretch of nucleotides (duplex-forming segment) that forms the other half of the dsRNA duplex of the protein-binding segment of the guide nucleic acid. In other words, a stretch of nucleotides of a crRNA-like molecule are complementary to and hybridize with a stretch of nucleotides of a tracrRNA-like molecule to form the dsRNA duplex of the protein-binding domain of the Cas9 guide RNA. As such, each targeter molecule can be said to have a corresponding activator molecule (which has a region that hybridizes with the targeter). The targeter molecule additionally provides the targeting segment. Thus, a targeter and an activator molecule (as a corresponding pair) hybridize to form a Cas9 guide RNA. The exact sequence of a given crRNA or tracrRNA molecule is characteristic of the species in which the RNA molecules are found. A subject dual Cas9 guide RNA can include any corresponding activator and targeter pair.

The term "activator" or "activator RNA" is used herein to mean a tracrRNA-like molecule (tracrRNA : "trans-acting CRISPR RNA") of a Cas9 dual guide RNA (and therefore of a Cas9 single guide RNA when the "activator" and the "targeter" are linked together by, *e.g.*, intervening nucleotides). Thus, for example, a Cas9 guide RNA (dgRNA or sgRNA) comprises an activator sequence *(e.g.,* a tracrRNA sequence). A tracr molecule (a tracrRNA) is a naturally existing molecule that hybridizes with a CRISPR RNA molecule (a crRNA) to form a Cas9 dual guide RNA. The term "activator" is used herein to encompass naturally existing tracrRNAs, but also to encompass tracrRNAs with modifications (*e.g.*, truncations, sequence variations, base modifications, backbone modifications, linkage modifications, *etc.)* where the activator retains at least one function of a tracrRNA *(e.g.,* contributes to the dsRNA duplex to which Cas9 protein binds). In some cases the activator provides one or more stem loops that can interact with Cas9 protein. An activator can be referred to as having a tracr sequence (tracrRNA sequence) and in some cases is a tracrRNA, but the term "activator" is not limited to naturally existing tracrRNAs.

The term "targeter" or "targeter RNA" is used herein to refer to a crRNA-like molecule (crRNA: "CRISPR RNA") of a Cas9 dual guide RNA (and therefore of a Cas9 single guide RNA when the "activator" and the "targeter" are linked together, *e.g.,* by intervening nucleotides). Thus, for example, a Cas9 guide RNA (dgRNA or sgRNA) comprises a targeting segment (which includes nucleotides that hybridize with (are complementary to) a target nucleic acid, and a duplex-forming segment *(e.g.,* a duplex forming segment of a crRNA, which can also be referred to as a crRNA repeat). Because the sequence of a targeting segment (the segment that hybridizes with a target sequence of a target nucleic acid) of a targeter is modified by a user to hybridize with a desired target nucleic acid, the sequence of a targeter will often be a non-naturally occurring sequence. However, the duplex-forming segment of a targeter (described in more detail below), which hybridizes with the duplex-forming segment of an activator, can include a naturally existing sequence *(e.g.,* can include the sequence of a duplex-forming segment of a naturally existing crRNA, which can also be referred to as a crRNA repeat). Thus, the term targeter is used herein to distinguish from naturally occurring crRNAs, despite the fact that part of a targeter *(e.g.,* the duplex-forming segment) often includes a naturally occurring sequence from a crRNA. However, the term "targeter" encompasses naturally occurring crRNAs.

A Cas9 guide RNA can also be said to include 3 parts: (i) a targeting sequence (a nucleotide sequence that hybridizes with a sequence of the target nucleic acid); (ii) an activator sequence (as described above)(in some cases, referred to as a tracr sequence); and (iii) a sequence that hybridizes to at least a portion of the activator sequence to form a double stranded duplex. A targeter has (i) and (iii); while an activator has (ii).

A Cas9 guide RNA *(e.g.,* a dual guide RNA or a single guide RNA) can be comprised of any corresponding activator and targeter pair. In some cases, the duplex forming segments can be swapped between the activator and the targeter. In other words, in some cases, the targeter includes a sequence of nucleotides from a duplex forming segment of a tracrRNA (which sequence would normally be part of an activator) while the activator includes a sequence of nucleotides from a duplex forming segment of a crRNA (which sequence would normally be part of a targeter).

As noted above, a targeter comprises both the targeting segment (single stranded) of the Cas9 guide RNA and a stretch ("duplex-forming segment") of nucleotides that forms one half of the dsRNA duplex of the protein-binding segment of the Cas9 guide RNA. A corresponding tracrRNA-like molecule (activator) comprises a stretch of nucleotides (a duplex-forming segment) that forms the other half of the dsRNA duplex of the protein-binding segment of the Cas9 guide RNA. In other words, a stretch of nucleotides of the targeter is complementary to and hybridizes with a stretch of nucleotides of the activator to form the dsRNA duplex of the protein-binding segment of a Cas9 guide RNA. As such, each targeter can be said to have a corresponding activator (which has a region that hybridizes with the targeter). The targeter molecule additionally provides the targeting segment. Thus, a targeter and an activator (as a corresponding pair) hybridize to form a Cas9 guide RNA. The particular sequence of a given naturally existing crRNA or tracrRNA molecule is characteristic of the species in which the RNA molecules are found. Examples of suitable activator and targeter are well known in the art.

A Cas9 guide RNA *(e.g.,* a dual guide RNA or a single guide RNA) can be comprised of any corresponding activator and targeter pair. Non-limiting examples of nucleotide sequences that can be included in a Cas9 guide RNA (dgRNA or sgRNA) include sequences set forth in SEQ ID NOs: 827-1075, or complements thereof. For example, in some cases, sequences from SEQ ID NOs: 827-957 (which are from tracrRNAs) or complements thereof, can pair with sequences from SEQ ID NOs: 964-1075 (which are from crRNAs), or complements thereof, to form a dsRNA duplex of a protein binding segment.

### Targeting segment of a Cas9 guide RNA

The first segment of a subject guide nucleic acid includes a guide sequence *(i.e.,* a targeting sequence)(a nucleotide sequence that is complementary to a sequence (a target site) in a target nucleic acid). In other words, the targeting segment of a subject guide nucleic acid can interact with a target nucleic acid *(e.g.,* double stranded DNA (dsDNA)) in a sequence-specific manner via hybridization (*i.e.,* base pairing). As such, the nucleotide sequence of the targeting segment may vary (depending on the target) and can determine the location within the target nucleic acid that the Cas9 guide RNA and the target nucleic acid will interact. The targeting segment of a Cas9 guide RNA can be modified *(e.g.,* by genetic engineering)/designed to hybridize to any desired sequence (target site) within a target nucleic acid *(e.g.,* a eukaryotic target nucleic acid such as genomic DNA).

The targeting segment can have a length of 7 or more nucleotides (nt) *(e.g.,* 8 or more, 9 or more, 10 or more, 12 or more, 15 or more, 20 or more, 25 or more, 30 or more, or 40 or more nucleotides). In some cases, the targeting segment can have a length of from 7 to 100 nucleotides (nt) (*e.g.,* from 7 to 80 nt, from 7 to 60 nt, from 7 to 40 nt, from 7 to 30 nt, from 7 to 25 nt, from 7 to 22 nt, from 7 to 20 nt, from 7 to 18 nt, from 8 to 80 nt, from 8 to 60 nt, from 8 to 40 nt, from 8 to 30 nt, from 8 to 25 nt, from 8 to 22 nt, from 8 to 20 nt, from 8 to 18 nt, from 10 to 100 nt, from 10 to 80 nt, from 10 to 60 nt, from 10 to 40 nt, from 10 to 30 nt, from 10 to 25 nt, from 10 to 22 nt, from 10 to 20 nt, from 10 to 18 nt, from 12 to 100 nt, from 12 to 80 nt, from 12 to 60 nt, from 12 to 40 nt, from 12 to 30 nt, from 12 to 25 nt, from 12 to 22 nt, from 12 to 20 nt, from 12 to 18 nt, from 14 to 100 nt, from 14 to 80 nt, from 14 to 60 nt, from 14 to 40 nt, from 14 to 30 nt, from 14 to 25 nt, from 14 to 22 nt, from 14 to 20 nt, from 14 to 18 nt, from 16 to 100 nt, from 16 to 80 nt, from 16 to 60 nt, from 16 to 40 nt, from 16 to 30 nt, from 16 to 25 nt, from 16 to 22 nt, from 16 to 20 nt, from 16 to 18 nt, from 18 to 100 nt, from 18 to 80 nt, from 18 to 60 nt, from 18 to 40 nt, from 18 to 30 nt, from 18 to 25 nt, from 18 to 22 nt, or from 18 to 20 nt)

The nucleotide sequence (the targeting sequence) of the targeting segment that is complementary to a nucleotide sequence (target site) of the target nucleic acid can have a length of 10 nt or more. For example, the targeting sequence of the targeting segment that is complementary to a target site of the target nucleic acid can have a length of 12 nt or more, 15 nt or more, 18 nt or more, 19 nt or more, or 20 nt or more. In some cases, the nucleotide sequence (the targeting sequence) of the targeting segment that is complementary to a nucleotide sequence (target site) of the target nucleic acid has a length of 12 nt or more. In some cases, the nucleotide sequence (the targeting sequence) of the targeting segment that is complementary to a nucleotide sequence (target site) of the target nucleic acid has a length of 18 nt or more.

For example, the targeting sequence of the targeting segment that is complementary to a target sequence of the target nucleic acid can have a length of from 10 to 100 nucleotides (nt) (*e.g.,* from 10 to 90 nt, from 10 to 75 nt, from 10 to 60 nt, from 10 to 50 nt, from 10 to 35 nt, from 10 to 30 nt, from 10 to 25 nt, from 10 to 22 nt, from 10 to 20 nt, from 12 to 100 nt, from 12 to 90 nt, from 12 to 75 nt, from 12 to 60 nt, from 12 to 50 nt, from 12 to 35 nt, from 12 to 30 nt, from 12 to 25 nt, from 12 to 22 nt, from 12 to 20 nt, from 15 to 100 nt, from 15 to 90 nt, from 15 to 75 nt, from 15 to 60 nt, from 15 to 50 nt, from 15 to 35 nt, from 15 to 30 nt, from 15 to 25 nt, from 15 to 22 nt, from 15 to 20 nt, from 17 to 100 nt, from 17 to 90 nt, from 17 to 75 nt, from 17 to 60 nt, from 17 to 50 nt, from 17 to 35 nt, from 17 to 30 nt, from 17 to 25 nt, from 17 to 22 nt, from 17 to 20 nt, from 18 to 100 nt, from 18 to 90 nt, from 18 to 75 nt, from 18 to 60 nt, from 18 to 50 nt, from 18 to 35 nt, from 18 to 30 nt, from 18 to 25 nt, from 18 to 22 nt, or from 18 to 20 nt). In some cases, the targeting sequence of the targeting segment that is complementary to a target sequence of the target nucleic acid has a length of from 15 nt to 30 nt. In some cases, the targeting sequence of the targeting segment that is complementary to a target sequence of the target nucleic acid has a length of from 15 nt to 25 nt. In some cases, the targeting sequence of the targeting segment that is complementary to a target sequence of the target nucleic acid has a length of from 18 nt to 30 nt. In some cases, the targeting sequence of the targeting segment that is complementary to a target sequence of the target nucleic acid has a length of from 18 nt to 25 nt. In some cases, the targeting sequence of the targeting segment that is complementary to a target sequence of the target nucleic acid has a length of from 18 nt to 22 nt. In some cases, the targeting sequence of the targeting segment that is complementary to a target site of the target nucleic acid is 20 nucleotides in length. In some cases, the targeting sequence of the targeting segment that is complementary to a target site of the target nucleic acid is 19 nucleotides in length.

The percent complementarity between the targeting sequence (guide sequence) of the targeting segment and the target site of the target nucleic acid can be 60% or more *(e.g.,* 65% or more, 70% or more, 75% or more, 80% or more, 85% or more, 90% or more, 95% or more, 97% or more, 98% or more, 99% or more, or 100%). In some cases, the percent complementarity between the targeting sequence of the targeting segment and the target site of the target nucleic acid is 100% over the seven contiguous 5'-most nucleotides of the target site of the target nucleic acid. In some cases, the percent complementarity between the targeting sequence of the targeting segment and the target site of the target nucleic acid is 60% or more over about 20 contiguous nucleotides. In some cases, the percent complementarity between the targeting sequence of the targeting segment and the target site of the target nucleic acid is 100% over the fourteen contiguous 5'-most nucleotides of the target site of the target nucleic acid and as low as 0% or more over the remainder. In such a case, the targeting sequence can be considered to be 14 nucleotides in length. In some cases, the percent complementarity between the targeting sequence of the targeting segment and the target site of the target nucleic acid is 100% over the seven contiguous 5'-most nucleotides of the target site of the target nucleic acid and as low as 0% or more over the remainder. In such a case, the targeting sequence can be considered to be 20 nucleotides in length.

In some cases, the percent complementarity between the targeting sequence of the targeting segment and the target site of the target nucleic acid is 100% over the 7 contiguous 5'-most nucleotides of the target site of the target nucleic acid (which can be complementary to the 3'-most nucleotides of the targeting sequence of the Cas9 guide RNA). In some cases, the percent complementarity between the targeting sequence of the targeting segment and the target site of the target nucleic acid is 100% over the 8 contiguous 5'-most nucleotides of the target site of the target nucleic acid (which can be complementary to the 3'-most nucleotides of the targeting sequence of the Cas9 guide RNA). In some cases, the percent complementarity between the targeting sequence of the targeting segment and the target site of the target nucleic acid is 100% over the 9 contiguous 5'-most nucleotides of the target site of the target nucleic acid (which can be complementary to the 3'-most nucleotides of the targeting sequence of the Cas9 guide RNA). In some cases, the percent complementarity between the targeting sequence of the targeting segment and the target site of the target nucleic acid is 100% over the 10 contiguous 5'-most nucleotides of the target site of the target nucleic acid (which can be complementary to the 3'-most nucleotides of the targeting sequence of the Cas9 guide RNA). In some cases, the percent complementarity between the targeting sequence of the targeting segment and the target site of the target nucleic acid is 100% over the 17 contiguous 5'-most nucleotides of the target site of the target nucleic acid (which can be complementary to the 3'-most nucleotides of the targeting sequence of the Cas9 guide RNA). In some cases, the percent complementarity between the targeting sequence of the targeting segment and the target site of the target nucleic acid is 100% over the 18 contiguous 5'-most nucleotides of the target site of the target nucleic acid (which can be complementary to the 3'-most nucleotides of the targeting sequence of the Cas9 guide RNA). In some cases, the percent complementarity between the targeting sequence of the targeting segment and the target site of the target nucleic acid is 60% or more *(e.g., e.g.,* 65% or more, 70% or more, 75% or more, 80% or more, 85% or more, 90% or more, 95% or more, 97% or more, 98% or more, 99% or more, or 100%) over about 20 contiguous nucleotides.

In some cases, the percent complementarity between the targeting sequence of the targeting segment and the target site of the target nucleic acid is 100% over the 7 contiguous 5'-most nucleotides of the target site of the target nucleic acid and as low as 0% or more over the remainder. In such a case, the targeting sequence can be considered to be 7 nucleotides in length. In some cases, the percent complementarity between the targeting sequence of the targeting segment and the target site of the target nucleic acid is 100% over the 8 contiguous 5'-most nucleotides of the target site of the target nucleic acid and as low as 0% or more over the remainder. In such a case, the targeting sequence can be considered to be 8 nucleotides in length. In some cases, the percent complementarity between the targeting sequence of the targeting segment and the target site of the target nucleic acid is 100% over the 9 contiguous 5'-most nucleotides of the target site of the target nucleic acid and as low as 0% or more over the remainder. In such a case, the targeting sequence can be considered to be 9 nucleotides in length. In some cases, the percent complementarity between the targeting sequence of the targeting segment and the target site of the target nucleic acid is 100% over the 10 contiguous 5'-most nucleotides of the target site of the target nucleic acid and as low as 0% or more over the remainder. In such a case, the targeting sequence can be considered to be 10 nucleotides in length. In some cases, the percent complementarity between the targeting sequence of the targeting segment and the target site of the target nucleic acid is 100% over the 11 contiguous 5'-most nucleotides of the target site of the target nucleic acid and as low as 0% or more over the remainder. In such a case, the targeting sequence can be considered to be 11 nucleotides in length. In some cases, the percent complementarity between the targeting sequence of the targeting segment and the target site of the target nucleic acid is 100% over the 12 contiguous 5'-most nucleotides of the target site of the target nucleic acid and as low as 0% or more over the remainder. In such a case, the targeting sequence can be considered to be 12 nucleotides in length. In some cases, the percent complementarity between the targeting sequence of the targeting segment and the target site of the target nucleic acid is 100% over the 13 contiguous 5'-most nucleotides of the target site of the target nucleic acid and as low as 0% or more over the remainder. In such a case, the targeting sequence can be considered to be 13 nucleotides in length. In some cases, the percent complementarity between the targeting sequence of the targeting segment and the target site of the target nucleic acid is 100% over the 14 contiguous 5'-most nucleotides of the target site of the target nucleic acid and as low as 0% or more over the remainder. In such a case, the targeting sequence can be considered to be 14 nucleotides in length. In some cases, the percent complementarity between the targeting sequence of the targeting segment and the target site of the target nucleic acid is 100% over the 17 contiguous 5'-most nucleotides of the target site of the target nucleic acid and as low as 0% or more over the remainder. In such a case, the targeting sequence can be considered to be 17 nucleotides in length. In some cases, the percent complementarity between the targeting sequence of the targeting segment and the target site of the target nucleic acid is 100% over the 18 contiguous 5'-most nucleotides of the target site of the target nucleic acid and as low as 0% or more over the remainder. In such a case, the targeting sequence can be considered to be 18 nucleotides in length.

### Protein-binding segment of a Cas9 guide RNA

The protein-binding segment of a subject Cas9 guide RNA interacts with a Cas9 protein. The Cas9 guide RNA guides the bound Cas9 protein to a specific nucleotide sequence within target nucleic acid via the above mentioned targeting segment. The protein-binding segment of a Cas9 guide RNA comprises two stretches of nucleotides that are complementary to one another and hybridize to form a double stranded RNA duplex (dsRNA duplex). Thus, the protein-binding segment includes a dsRNA duplex. In some cases, the protein-binding segment also includes stem loop 1 (the "nexus") of a Cas9 guide RNA. For example, in some cases, the activator of a Cas9 guide RNA (dgRNA or sgRNA) includes (i) a duplex forming segment that contributes to the dsRNA duplex of the protein-binding segment; and (ii) nucleotides 3' of the duplex forming segment, e.g., that form stem loop 1 (the "nexus"). For example, in some cases, the protein-binding segment includes stem loop 1 (the "nexus") of a Cas9 guide RNA. In some cases, the protein-binding segment includes 5 or more nucleotides (nt) *(e.g.,* 6 or more, 7 or more, 8 or more, 9 or more, 10 or more, 11 or more, 12 or more, 15 or more, 20 or more, 30 or more, 40 or more, 50 or more, 60 or more, 70 or more, 75 or more, or 80 or more nt) 3' of the dsRNA duplex (where 3' is relative to the duplex-forming segment of the activator sequence).

The dsRNA duplex of the guide RNA (sgRNA or dgRNA) that forms between the activator and targeter is sometimes referred to herein as the "stem loop". In addition, the activator (activator RNA, tracrRNA) of many naturally existing Cas9 guide RNAs *(e.g.,* S. *pygogenes* guide RNAs) has 3 stem loops (3 hairpins) that are 3' of the duplex-forming segment of the activator. The closest stem loop to the duplex-forming segment of the activator (3' of the duplex forming segment) is called "stem loop 1" (and is also referred to herein as the "nexus"); the next stem loop is called "stem loop 2" (and is also referred to herein as the "hairpin 1"); and the next stem loop is called "stem loop 3" (and is also referred to herein as the "hairpin 2").

In some cases, a Cas9 guide RNA (sgRNA or dgRNA) *(e.g.,* a full length Cas9 guide RNA) has stem loops 1, 2, and 3. In some cases, an activator (of a Cas9 guide RNA) has stem loop 1, but does not have stem loop 2 and does not have stem loop 3. In some cases, an activator (of a Cas9 guide RNA) has stem loop 1 and stem loop 2, but does not have stem loop 3. In some cases, an activator (of a Cas9 guide RNA) has stem loops 1, 2, and 3.

In some cases, the activator (e.g., tracr sequence) of a Cas9 guide RNA (dgRNA or sgRNA) includes (i) a duplex forming segment that contributes to the dsRNA duplex of the protein-binding segment; and (ii) a stretch of nucleotides (e.g., referred to herein as a 3' tail) 3' of the duplex forming segment. In some cases, the additional nucleotides 3' of the duplex forming segment form stem loop 1. In some cases, the activator *(e.g.,* tracr sequence) of a Cas9 guide RNA (dgRNA or sgRNA) includes (i) a duplex forming segment that contributes to the dsRNA duplex of the protein-binding segment; and (ii) 5 or more nucleotides *(e.g.,* 6 or more, 7 or more, 8 or more, 9 or more, 10 or more, 11 or more, 12 or more, 13 or more, 14 or more, 15 or more, 20 or more, 25 or more, 30 or more, 35 or more, 40 or more, 45 or more, 50 or more, 60 or more, 70 or more, or 75 or more nucleotides) 3' of the duplex forming segment. In some cases, the activator (activator RNA) of a Cas9 guide RNA (dgRNA or sgRNA) includes (i) a duplex forming segment that contributes to the dsRNA duplex of the protein-binding segment; and (ii) 5 or more nucleotides *(e.g.,* 6 or more, 7 or more, 8 or more, 9 or more, 10 or more, 11 or more, 12 or more, 13 or more, 14 or more, 15 or more, 20 or more, 25 or more, 30 or more, 35 or more, 40 or more, 45 or more, 50 or more, 60 or more, 70 or more, or 75 or more nucleotides) 3' of the duplex forming segment.

In some cases, the activator (e.g., tracr sequence) of a Cas9 guide RNA (dgRNA or sgRNA) includes (i) a duplex forming segment that contributes to the dsRNA duplex of the protein-binding segment; and (ii) a stretch of nucleotides (e.g., referred to herein as a 3' tail) 3' of the duplex forming segment. In some cases, the stretch of nucleotides 3' of the duplex forming segment has a length in a range of from 5 to 200 nucleotides (nt) *(e.g.,* from 5 to 150 nt, from 5 to 130 nt, from 5 to 120 nt, from 5 to 100 nt, from 5 to 80 nt, from 10 to 200 nt, from 10 to 150 nt, from 10 to 130 nt, from 10 to 120 nt, from 10 to 100 nt, from 10 to 80 nt, from 12 to 200 nt, from 12 to 150 nt, from 12 to 130 nt, from 12 to 120 nt, from 12 to 100 nt, from 12 to 80 nt, from 15 to 200 nt, from 15 to 150 nt, from 15 to 130 nt, from 15 to 120 nt, from 15 to 100 nt, from 15 to 80 nt, from 20 to 200 nt, from 20 to 150 nt, from 20 to 130 nt, from 20 to 120 nt, from 20 to 100 nt, from 20 to 80 nt, from 30 to 200 nt, from 30 to 150 nt, from 30 to 130 nt, from 30 to 120 nt, from 30 to 100 nt, or from 30 to 80 nt). In some cases, the nucleotides of the 3' tail of an activator RNA are wild type sequences. Although a number of different alternative sequences can be used, an example Cas9 single guide RNA (based on crRNA and tracrRNA from S. *pyogenes,* where the dsRNA duplex of the protein-binding segment is truncated relative to the dsRNA duplex present in the wild type dual guide RNA) can include the sequence set forth in SEQ ID NO:958 (This example sequence does not include the guide sequence. The guide sequence, which varies depending on the target, would be 5' of this example sequence. The activator in this example is 66 nucleotides long).

Examples of various Cas9 proteins and Cas9 guide RNAs (as well as information regarding requirements related to protospacer adjacent motif (PAM) sequences present in targeted nucleic acids) can be found in the art, for example, see Jinek et al., Science. 2012 Aug 17;337(6096):816-21; Chylinski et al., RNA Biol. 2013 May;10(5):726-37; Ma et al., Biomed Res Int. 2013;2013:270805; Hou et al., Proc Natl Acad Sci U S A. 2013 Sep 24;110(39):15644-9; Jinek et al., Elife. 2013;2:e00471; Pattanayak et al., Nat Biotechnol. 2013 Sep;31(9):839-43; Qi et al, Cell. 2013 Feb 28;152(5):1173-83; Wang et al., Cell. 2013 May 9;153(4):910-8; Auer et. al., Genome Res. 2013 Oct 31; Chen et. al., Nucleic Acids Res. 2013 Nov 1;41(20):e19; Cheng et. al., Cell Res. 2013 Oct;23(10):1163-71; Cho et. al., Genetics. 2013 Nov;195(3):1177-80; DiCarlo et al., Nucleic Acids Res. 2013 Apr;41(7):4336-43; Dickinson et. al., Nat Methods. 2013 Oct;10(10):1028-34; Ebina et. al., Sci Rep. 2013;3:2510; Fujii et. al, Nucleic Acids Res. 2013 Nov 1;41(20):e187; Hu et. al., Cell Res. 2013 Nov;23(11):1322-5; Jiang et. al., Nucleic Acids Res. 2013 Nov 1;41(20):e188; Larson et. al., Nat Protoc. 2013 Nov;8(11):2180-96; Mali et. at., Nat Methods. 2013 Oct;10(10):957-63; Nakayama et. al., Genesis. 2013 Dec;51(12):835-43; Ran et. al., Nat Protoc. 2013 Nov;8(11):2281-308; Ran et. al., Cell. 2013 Sep 12;154(6):1380-9; Upadhyay et. al., G3 (Bethesda). 2013 Dec 9;3(12):2233-8; Walsh et. al., Proc Natl Acad Sci USA. 2013 Sep 24;110(39):15514-5; Xie et. al., Mol Plant. 2013 Oct 9; Yang et. al., Cell. 2013 Sep 12;154(6):1370-9; Briner et al., Mol Cell. 2014 Oct 23;56(2):333-9; and U.S. patents and patent applications: 8,906,616; 8,895,308; 8,889,418; 8,889,356; 8,871,445; 8,865,406; 8,795,965; 8,771,945; 8,697,359; 20140068797; 20140170753; 20140179006; 20140179770; 20140186843; 20140186919; 20140186958; 20140189896; 20140227787; 20140234972; 20140242664; 20140242699; 20140242700; 20140242702; 20140248702; 20140256046; 20140273037; 20140273226; 20140273230; 20140273231; 20140273232; 20140273233; 20140273234; 20140273235; 20140287938; 20140295556; 20140295557; 20140298547; 20140304853; 20140309487; 20140310828; 20140310830; 20140315985; 20140335063; 20140335620; 20140342456; 20140342457; 20140342458; 20140349400; 20140349405; 20140356867; 20140356956; 20140356958; 20140356959; 20140357523; 20140357530; 20140364333; and 20140377868.

### Guide RNAs corresponding to type V and type VI CRISPR/Cas endonucleases (e.g., Cpf1 Guide RNA)

A guide RNA that binds to a type V or type VI CRISPR/Cas protein *(e.g.,* Cpf1, C2c1, C2c2, C2c3), and targets the complex to a specific location within a target nucleic acid is referred to herein generally as a "type V or type VI CRISPR/Cas guide RNA" . An example of a more specific term is a "Cpfl guide RNA."

A type V or type VI CRISPR/Cas guide RNA *(e.g.,* cpf1 guide RNA) can have a total length of from 30 nucleotides (nt) to 200 nt, *e.g.,* from 30 nt to 180 nt, from 30 nt to 160 nt, from 30 nt to 150 nt, from 30 nt to 125 nt, from 30 nt to 100 nt, from 30 nt to 90 nt, from 30 nt to 80 nt, from 30 nt to 70 nt, from 30 nt to 60 nt, from 30 nt to 50 nt, from 50 nt to 200 nt, from 50 nt to 180 nt, from 50 nt to 160 nt, from 50 nt to 150 nt, from 50 nt to 125 nt, from 50 nt to 100 nt, from 50 nt to 90 nt, from 50 nt to 80 nt, from 50 nt to 70 nt, from 50 nt to 60 nt, from 70 nt to 200 nt, from 70 nt to 180 nt, from 70 nt to 160 nt, from 70 nt to 150 nt, from 70 nt to 125 nt, from 70 nt to 100 nt, from 70 nt to 90 nt, or from 70 nt to 80 nt). In some cases, a type V or type VI CRISPR/Cas guide RNA *(e.g.,* cpf1 guide RNA) has a total length of at least 30 nt *(e.g.,* at least 40 nt, at least 50 nt, at least 60 nt, at least 70 nt, at least 80 nt, at least 90 nt, at least 100 nt, or at least 120 nt).

In some cases, a Cpfl guide RNA has a total length of 35 nt, 36 nt, 37 nt, 38 nt, 39 nt, 40 nt, 41 nt, 42 nt, 43 nt, 44 nt, 45 nt, 46 nt, 47 nt, 48 nt, 49 nt, or 50 nt.

Like a Cas9 guide RNA, a type V or type VI CRISPR/Cas guide RNA *(e.g.,* cpf1 guide RNA) can include a target nucleic acid-binding segment and a duplex-forming region *(e.g.,* in some cases formed from two duplex-forming segments, *i.e.,* two stretches of nucleotides that hybridize to one another to form a duplex)

The target nucleic acid-binding segment of a type V or type VI CRISPR/Cas guide RNA *(e.g.,* cpf1 guide RNA) can have a length of from 15 nt to 30 nt, *e.g.,* 15 nt, 16 nt, 17 nt, 18 nt, 19 nt, 20 nt, 21 nt, 22 nt, 23 nt, 24 nt, 25 nt, 26 nt, 27 nt, 28 nt, 29 nt, or 30 nt. In some cases, the target nucleic acid-binding segment has a length of 23 nt. In some cases, the target nucleic acid-binding segment has a length of 24 nt. In some cases, the target nucleic acid-binding segment has a length of 25 nt.

The guide sequence of a type V or type VI CRISPR/Cas guide RNA *(e.g.,* cpf1 guide RNA) can have a length of from 15 nt to 30 nt *(e.g.,* 15 to 25 nt, 15 to 24 nt, 15 to 23 nt, 15 to 22 nt, 15 to 21 nt, 15 to 20 nt, 15 to 19 nt, 15 to 18 nt,17 to 30 nt, 17 to 25 nt, 17 to 24 nt, 17 to 23 nt, 17 to 22 nt, 17 to 21 nt, 17 to 20 nt, 17 to 19 nt, 17 to 18 nt, 18 to 30 nt, 18 to 25 nt, 18 to 24 nt, 18 to 23 nt, 18 to 22 nt, 18 to 21 nt, 18 to 20 nt, 18 to 19 nt, 19 to 30 nt, 19 to 25 nt, 19 to 24 nt, 19 to 23 nt, 19 to 22 nt, 19 to 21 nt, 19 to 20 nt, 20 to 30 nt, 20 to 25 nt, 20 to 24 nt, 20 to 23 nt, 20 to 22 nt, 20 to 21 nt, 15 nt, 16 nt, 17 nt, 18 nt, 19 nt, 20 nt, 21 nt, 22 nt, 23 nt, 24 nt, 25 nt, 26 nt, 27 nt, 28 nt, 29 nt, or 30 nt). In some cases, the guide sequence has a length of 17 nt. In some cases, the guide sequence has a length of 18 nt. In some cases, the guide sequence has a length of 19 nt. In some cases, the guide sequence has a length of 20 nt. In some cases, the guide sequence has a length of 21 nt. In some cases, the guide sequence has a length of 22 nt. In some cases, the guide sequence has a length of 23 nt. In some cases, the guide sequence has a length of 24 nt.

The guide sequence of a type V or type VI CRISPR/Cas guide RNA *(e.g.,* cpf1 guide RNA) can have 100% complementarity with a corresponding length of target nucleic acid sequence. The guide sequence can have less than 100% complementarity with a corresponding length of target nucleic acid sequence. For example, the guide sequence of a type V or type VI CRISPR/Cas guide RNA *(e.g.,* cpf1 guide RNA) can have 1, 2, 3, 4, or 5 nucleotides that are not complementary to the target nucleic acid sequence. For example, in some cases, where a guide sequence has a length of 25 nucleotides, and the target nucleic acid sequence has a length of 25 nucleotides, in some cases, the target nucleic acid-binding segment has 100% complementarity to the target nucleic acid sequence. As another example, in some cases, where a guide sequence has a length of 25 nucleotides, and the target nucleic acid sequence has a length of 25 nucleotides, in some cases, the target nucleic acid-binding segment has 1 non-complementary nucleotide and 24 complementary nucleotides with the target nucleic acid sequence. As another example, in some cases, where a guide sequence has a length of 25 nucleotides, and the target nucleic acid sequence has a length of 25 nucleotides, in some cases, the target nucleic acid-binding segment has 2 non-complementary nucleotides and 23 complementary nucleotides with the target nucleic acid sequence.

The duplex-forming segment of a type V or type VI CRISPR/Cas guide RNA *(e.g.,* cpf1 guide RNA) *(e.g.,* of a targeter RNA or an activator RNA) can have a length of from 15 nt to 25 nt *(e.g.,* 15 nt, 16 nt, 17 nt, 18 nt, 19 nt, 20 nt, 21 nt, 22 nt, 23 nt, 24 nt, or 25 nt).

The RNA duplex of a type V or type VI CRISPR/Cas guide RNA *(e.g.,* cpf1 guide RNA) can have a length of from 5 base pairs (bp) to 40 bp *(e.g.,* from 5 to 35 bp, 5 to 30 bp, 5 to 25 bp, 5 to 20 bp, 5 to 15 bp, 5-12 bp, 5-10 bp, 5-8 bp, 6 to 40 bp, 6 to 35 bp, 6 to 30 bp, 6 to 25 bp, 6 to 20 bp, 6 to 15 bp, 6 to 12 bp, 6 to 10 bp, 6 to 8 bp, 7 to 40 bp, 7 to 35 bp, 7 to 30 bp, 7 to 25 bp, 7 to 20 bp, 7 to 15 bp, 7 to 12 bp, 7 to 10 bp, 8 to 40 bp, 8 to 35 bp, 8 to 30 bp, 8 to 25 bp, 8 to 20 bp, 8 to 15 bp, 8 to 12 bp, 8 to 10 bp, 9 to 40 bp, 9 to 35 bp, 9 to 30 bp, 9 to 25 bp, 9 to 20 bp, 9 to 15 bp, 9 to 12 bp, 9 to 10 bp, 10 to 40 bp, 10 to 35 bp, 10 to 30 bp, 10 to 25 bp, 10 to 20 bp, 10 to 15 bp, or 10 to 12bp).

As an example, a duplex-forming segment of a Cpfl guide RNA can comprise a nucleotide sequence selected from (5' to 3'): AAUUUCUACUGUUGUAGAU (SEQ ID NO:1093), AAUUUCUGCUGUUGCAGAU (SEQ ID NO:1094), AAUUUCCACUGUUGUGGAU (SEQ ID NO:1095), AAUUCCUACUGUUGUAGGU (SEQ ID NO:1096), AAUUUCUACUAUUGUAGAU (SEQ ID NO:1097), AAUUUCUACUGCUGUAGAU (SEQ ID NO:1098), AAUUUCUACUUUGUAGAU (SEQ ID NO:1099), and AAUUUCUACUUGUAGAU (SEQ ID NO:1100). The guide sequence can then follow (5' to 3') the duplex forming segment.

A non-limiting example of an activator RNA *(e.g.,* tracrRNA) of a C2c1 guide RNA (dual guide or single guide) is an RNA that includes the nucleotide sequence GAAUUUUUCAACGGGUGUGCCAAUGGCCACUUUCCAGGUGGCAAAGCCCGUUG AGCUUCUCAAAAAG (SEQ ID NO:1101). In some cases, a C2c1 guide RNA (dual guide or single guide) is an RNA that includes the nucleotide sequence In some cases, a C2c1 guide RNA (dual guide or single guide) is an RNA that includes the nucleotide sequence GUCUAGAGGACAGAAUUUUUCAACGGGUGUGCCAAUGGCCA CUUUCCAGGUGGCAAAGCCCGUUGAGCUUCUCAAAAAG (SEQ ID NO:1102). In some cases, a C2c1 guide RNA (dual guide or single guide) is an RNA that includes the nucleotide sequence UCUAGAGGACAGAAUUUUUCAACGGGUGUGCCA AUGGCCACU UUCCAGGUGGCAAAGCCCGUUGAGCUUCUCAAAAAG (SEQ ID NO:1103). A non-limiting example of an activator RNA *(e.g.,* tracrRNA) of a C2c1 guide RNA (dual guide or single guide) is an RNA that includes the nucleotide sequence ACUUUCCAGGCAAAGCCCGUUGAGCUUCUCAAAAAG (SEQ ID NO:1104). In some cases, a duplex forming segment of a C2c1 guide RNA (dual guide or single guide) of an activator RNA *(e.g.,* tracrRNA) includes the nucleotide sequence AGCUUCUCA (SEQ ID NO:1105) or the nucleotide sequence GCUUCUCA (SEQ ID NO:1106) (the duplex forming segment from a naturally existing tracrRNA.

A non-limiting example of a targeter RNA *(e.g.,* crRNA) of a C2c1 guide RNA (dual guide or single guide) is an RNA with the nucleotide sequence CUGAGAAGUGGCACNNNNNNNNNNNNNNNNNNNN (SEQ ID NO:1107), where the Ns represent the guide sequence, which will vary depending on the target sequence, and although 20 Ns are depicted a range of different lengths are acceptable. In some cases, a duplex forming segment of a C2c1 guide RNA (dual guide or single guide) of a targeter RNA *(e.g.,* crRNA) includes the nucleotide sequence CUGAGAAGUGGCAC (SEQ ID NO:1108) or includes the nucleotide sequence CUGAGAAGU (SEQ ID NO:1109) or includes the nucleotide sequence UGAGAAGUGGCAC (SEQ ID NO:1110) or includes the nucleotide sequence UGAGAAGU (SEQ ID NO:1111).

Examples and guidance related to type V or type VI CRISPR/Cas endonucleases and guide RNAs (as well as information regarding requirements related to protospacer adjacent motif (PAM) sequences present in targeted nucleic acids) can be found in the art, for example, see Zetsche et al, Cell. 2015 Oct 22;163(3):759-71; Makarova et al, Nat Rev Microbiol. 2015 Nov;13(11):722-36; and Shmakov et al., Mol Cell. 2015 Nov 5;60(3):385-97.

### Target genomic DNA

A target nucleic acid *(e.g.,* target genomic DNA) is located within a stem cell, where suitable stem cells are described above. A target genomic DNA can be any genomic DNA in which the sequence is to be modified, *e.g.,* by substitution and/or insertion and/or deletion of one or more nucleotides present in the target genomic DNA.

Target genes (target genomic DNA) include those genes involved in various diseases or conditions. In some cases, the target genomic DNA is mutated, such that it encodes a non-functional polypeptide, or such that a polypeptide encoded by the target genomic DNA is not synthesized in any detectable amount, or such that a polypeptide encoded by the target genomic DNA is synthesized in a lower than normal amount, such that an individual having the mutation has a disease. Such diseases include, but are not limited to, achondroplasia, achromatopsia, acid maltase deficiency, adenosine deaminase deficiency, adrenoleukodystrophy, aicardi syndrome, alpha-1 antitrypsin deficiency, alpha-thalassemia, androgen insensitivity syndrome, apert syndrome, arrhythmogenic right ventricular, dysplasia, ataxia telangictasia, barth syndrome, beta-thalassemia, blue rubber bleb nevus syndrome, canavan disease, chronic granulomatous diseases (CGD), cri du chat syndrome, Crigler-Najjer Syndrome, cystic fibrosis, dercum's disease, ectodermal dysplasia, fanconi anemia, fibrodysplasia ossificans progressive, fragile X syndrome, galactosemis, Gaucher's disease, generalized gangliosidoses (e.g., GM1), Glycogen Storage Disease Type IV, hemochromatosis, the hemoglobin C mutation in the 6th codon of beta-globin (HbC), hemophilia, Huntington's disease, Hurler Syndrome, hypophosphatasia, Klinefelter syndrome, Krabbes Disease, Langer-Giedion Syndrome, leukocyte adhesion deficiency (LAD, OMIM No. 116920), leukodystrophy, long QT syndrome, Marfan syndrome, Moebius syndrome, mucopolysaccharidosis (MPS), nail patella syndrome, nephrogenic diabetes insipdius, neurofibromatosis, Neimann-Pick disease, osteogenesis imperfecta, porphyria, Prader-Willi syndrome, progeria, Proteus syndrome, retinoblastoma, Rett syndrome, Rubinstein-Taybi syndrome, Sanfilippo syndrome, severe combined immunodeficiency (SCID), Shwachman syndrome, sickle cell disease (sickle cell anemia), Smith-Magenis syndrome, Stickler syndrome, Tay-Sachs disease, Thrombocytopenia Absent Radius (TAR) syndrome, Treacher Collins syndrome, trisomy, tuberous sclerosis, Turner's syndrome, urea cycle disorder, von Hippel-Landau disease, Waardenburg syndrome, Williams syndrome, Wilson's disease, Wiskott-Aldrich syndrome, and X-linked lymphoproliferative syndrome. Other such diseases include, *e.g.,* acquired immunodeficiencies, lysosomal storage diseases *(e.g.,* Gaucher's disease, GM1, Fabry disease and Tay-Sachs disease), mucopolysaccahidosis *(e.g.,* Hunter's disease, Hurler's disease), hemoglobinopathies *(e.g.,* sickle cell diseases, HbC, α-thalassemia, β-thalassemia) and hemophilias.

For example, in some cases, the target genomic DNA comprises a mutation that gives rise to a trinucleotide repeat disease. Exemplary trinucleotide repeat diseases and target genes involved in trinucleotide repeat diseases Trinucleotide Repeat Diseases Gene DRPLA (Dentatorubropallidoluysian atrophy) ATN1 or DRPLA HD (Huntington's disease) HTT (Huntingtin) SBMA (Spinobulbar muscular atrophy or Androgen receptor on the Kennedy disease) X chromosome. SCA1 (Spinocerebellar ataxia Type 1) ATXN1 SCA2 (Spinocerebellar ataxia Type 2) ATXN2 SCA3 (Spinocerebellar ataxia Type 3 or ATXN3 Machado-Joseph disease) SCA6 (Spinocerebellar ataxia Type 6) CACNA1A SCA7 (Spinocerebellar ataxia Type 7) ATXN7 SCA17 (Spinocerebellar ataxia Type 17) TBP FRAXA (Fragile X syndrome) FMR1, on the X- chromosome FXTAS (Fragile X-associated tremor/ FMR1, on the X- ataxia syndrome) chromosome FRAXE (Fragile XE mental retardation) AFF2 or FMR2, on the X-chromosome FRDA (Friedreich's ataxia) FXN or X25, (frataxin- reduced expression) DM (Myotonic dystrophy) DMPK SCA8 (Spinocerebellar ataxia Type 8) OSCA or SCA8 SCA12 (Spinocerebellar ataxia Type 12) PPP2R2B or SCA12.

For example, in some cases, a suitable target genomic DNA is a β-globin gene, *e.g.,* a β-globin gene with a sickle cell mutation. As another example, a suitable target genomic DNA is a Huntington's locus, *e.g.,* an *HTT* gene, where the *HTT* gene comprises a mutation *(e.g.,* a CAG repeat expansion comprising more than 35 CAG repeats) that gives rise to Huntington's disease. As another example, a suitable target genomic DNA is an adenosine deaminase gene that comprises a mutation that gives rise to severe combined immunodeficiency. As another example, a suitable target genomic DNA is a BCL11A gene comprising a mutation associated with control of the gamma-globin genes. As another example, a suitable target genomic DNA is a BCL11a enhancer.

### Donor polynucleotide

In some cases, a genome editing composition comprises a donor template nucleic acid ("donor polynucleotide"). In some cases, a method of the present disclosure comprises contacting the target DNA with a donor polynucleotide, wherein the donor polynucleotide, a portion of the donor polynucleotide, a copy of the donor polynucleotide, or a portion of a copy of the donor polynucleotide integrates into the target DNA *(e.g.,* via homology-directed repair). In some cases, the method does not comprise contacting the cell with a donor polynucleotide (e.g., resulting in non-homologous end-joining). A donor poly nucleotide can be introduced into a target cell using any convenient technique for introducing nucleic acids into cells.

When it is desirable to insert a polynucleotide sequence into a target DNA sequence, a polynucleotide comprising a donor sequence to be inserted is provided to the cell *(e.g.,* the target DNA is contacted with a donor polynucleotide in addition to a genome editing composition (e.g., a genome editing endonuclease; or a genome-editing endonuclease and a guide RNA). By a "donor sequence" or "donor polynucleotide" it is meant a nucleic acid sequence to be inserted at the cleavage site induced by a genome-editing endonuclease. A suitable donor polynucleotide can be single stranded or double stranded. For example, in some cases, a donor polynucleotide is single stranded *(e.g.,* in some cases can be referred to as an oligonucleotide), and in some cases a donor polynucleotide is double stranded *(e.g.,* in some cases can be include two separate oligonucleotides that are hybridized). The donor polynucleotide will contain sufficient homology to a genomic sequence at the cleavage site, *e.g.,* 70%, 80%, 85%, 90%, 95%, or 100% homology with the nucleotide sequences flanking the cleavage site, *e.g.,* within 100 bases or less *(e.g.,* 50 bases or less of the cleavage site, *e.g.,* within 30 bases, within 15 bases, within 10 bases, within 5 bases, or immediately flanking the cleavage site), to support homology-directed repair between it and the genomic sequence to which it bears homology. Approximately 25 nucleotides (nt) or more *(e.g.,* 30 nt or more, 40 nt or more, 50 nt or more, 60 nt or more, 70 nt or more, 80 nt or more, 90 nt or more, 100 nt or more, 150 nt or more, 200 nt or more, *etc.)* of sequence homology between a donor and a genomic sequence (or any integral value between 10 and 200 nucleotides, or more) can support homology-directed repair. For example, in some cases, the 5' and/or the 3' flanking homology arm *(e.g.,* in some cases both of the flanking homology arms) of a donor polynucleotide can be 30 nucleotides (nt) or more in length *(e.g.,* 40 nt or more, 50 nt or more, 60 nt or more, 70 nt or more, 80 nt or more, 90 nt or more, 100 nt or more, *etc.).* For example, in some cases, the 5' and/or the 3' flanking homology arm *(e.g.,* in some cases both of the flanking homology arms) of a donor polynucleotide can have a length in a range of from 30 nt to 500 nt *(e.g.,* 30 nt to 400 nt, 30 nt to 350 nt, 30 nt to 300 nt, 30 nt to 250 nt, 30 nt to 200 nt, 30 nt to 150 nt, 30 nt to 100 nt, 30 nt to 90 nt, 30 nt to 80 nt, 50 nt to 400 nt, 50 nt to 350 nt, 50 nt to 300 nt, 50 nt to 250 nt, 50 nt to 200 nt, 50 nt to 150 nt, 50 nt to 100 nt, 50 nt to 90 nt, 50 nt to 80 nt, 60 nt to 400 nt, 60 nt to 350 nt, 60 nt to 300 nt, 60 nt to 250 nt, 60 nt to 200 nt, 60 nt to 150 nt, 60 nt to 100 nt, 60 nt to 90 nt, 60 nt to 80 nt).

Donor sequences can be of any length, *e.g.,* 10 nucleotides or more, 50 nucleotides or more, 100 nucleotides or more, 250 nucleotides or more, 500 nucleotides or more, 1000 nucleotides or more, 5000 nucleotides or more, *etc.*

The donor sequence is typically not identical to the genomic sequence that it replaces. Rather, the donor sequence may contain at least one or more single base changes, insertions, deletions, inversions or rearrangements with respect to the genomic sequence, so long as sufficient homology is present to support homology-directed repair. In some embodiments, the donor sequence comprises a non-homologous sequence flanked by two regions of homology, such that homology-directed repair between the target DNA region and the two flanking sequences results in insertion of the non-homologous sequence at the target region. Donor sequences may also comprise a vector backbone containing sequences that are not homologous to the DNA region of interest and that are not intended for insertion into the DNA region of interest. Generally, the homologous region(s) of a donor sequence will have at least 50% sequence identity to a genomic sequence with which recombination is desired. In certain embodiments, 60%, 70%, 80%, 90%, 95%, 98%, 99%, or 99.9% sequence identity is present. Any value between 1% and 100% sequence identity can be present, depending upon the length of the donor polynucleotide."

In some cases, a donor polynucleotide is delivered to the cell (introduced into a cell) as part of viral vector *(e.g.,* an adeno-associated virus (AAV) vector; a lentiviral vector; *etc.).* For example a viral DNA *(e.g.,* AAV DNA) can include a donor polynucleotide sequence (donor sequence) *(e.g.,* a virus, *e.g.,* AAV, can include a DNA molecule that includes a donor polynucleotide sequence). In some cases, a donor polynucleotide is introduced into a cell as a virus *(e.g.,* an AAV, *e.g.,* the donor polynucleotide sequence is present as part of the viral DNA, *e.g.,* AAV DNA) and the genome-editing endonuclease *(e.g.,* ZFN; Cas9 protein; *etc.)* and, where applicable, a guide RNA are delivered by a different route. For example, in some cases, a donor polynucleotide is introduced into a cell as a virus *(e.g.,* an AAV, *e.g.,* the donor polynucleotide sequence is present as part of the viral DNA, *e.g.,* AAV DNA) and a Cas9 protein and Cas9 guide RNA are delivered as part of a separate expression vector. In some cases, a donor polynucleotide is introduced into a cell as a virus *(e.g.,* an AAV, *e.g.,* the donor polynucleotide sequence is present as part of the viral DNA, *e.g.,* AAV DNA) and a Cas9 protein and Cas9 guide RNA are delivered as part of a ribonucleoprotein complex (RNP). In some cases: (i) a donor polynucleotide is introduced into a cell as a virus *(e.g.,* an AAV, *e.g.,* the donor polynucleotide sequence is present as part of the viral DNA, *e.g.,* AAV DNA), (ii) a Cas9 guide RNA is delivered as either an RNA or DNA encoding the RNA, and (iii) a Cas9 protein is delivered as a protein or as a nucleic acid encoding the protein *(e.g.,* RNA or DNA).

In some cases, a recombinant viral vector *(e.g.,* a recombinant AAV vector) comprising a donor polynucleotide is introduced into a cell before a Cas9-guide RNA RNP is introduced into the cell. For example, in some cases, a recombinant viral vector *(e.g.,* a recombinant AAV vector) comprising a donor polynucleotide is introduced into a cell from 2 hours to 72 hours *(e.g.,* from 2 hours to 4 hours, from 4 hours to 8 hours, from 8 hours to 12 hours, from 12 hours to 24 hours, from 24 hours to 48 hours, or from 48 hours to 72 hours) before the Cas9-guide RNA RNP is introduced into the cell.

### EXAMPLES

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used *(e.g.,* amounts, temperature, *etc.)* but some experimental errors and deviations should be accounted for. Unless indicated otherwise, parts are parts by weight, molecular weight is weight average molecular weight, temperature is in degrees Celsius, and pressure is at or near atmospheric. Standard abbreviations may be used, *e.g.,* bp, base pair(s); kb, kilobase(s); pl, picoliter(s); s or sec, second(s); min, minute(s); h or hr, hour(s); aa, amino acid(s); kb, kilobase(s); bp, base pair(s); nt, nucleotide(s); i.m., intramuscular(ly); i.p., intraperitoneal(ly); s.c., subcutaneous(ly); and the like.

### Example 1

Site-specific gene correction of the point mutation that underlies sickle cell disease (SCD) constitutes a precise strategy to generate a life-long source of gene-corrected erythrocytes. Previous work has shown that efficient gene correction can be achieved in CD34⁺ stem and progenitor cells; nevertheless, levels of HDR-mediated gene modification in long-term reconstituting hematopoietic stem cells (HSCs) remain low. The main objective of this study was to identify and characterize the mechanisms which underlie decreased HDR efficacy in primitive HSCs, by comprehensive evaluation of the cellular and molecular mechanisms that govern site-specific gene modification in mature vs. primitive human hematopoietic stem and progenitor cell populations. Zinc finger nucleases (ZFNs) designed to target the sickle mutation in the human β-globin gene that causes SCD were used. The ZFNs create double-strand breaks that induce cellular DNA damage repair pathways which results in DNA repair either through non-homologous-end-joining (NHEJ) or homology-directed repair (HDR) when co-delivered with a donor template. In immunophenotypically defined human cell populations: HSCs (CD34⁺/CD38⁻/CD90⁺CD45RA⁻); multipotent progenitors (MPPs) (CD34⁺/CD38⁻/CD45RA⁻/CD90⁻); and progenitor cells (CD34⁺/CD38⁺), efficiency of delivery and time-course of expression mRNA encoding GFP or ZFN, efficacy of two homologous donors, cell cycle status, gene expression of key HDR genes and cytotoxic responses to the treatment, were assessed. In concordance with previous findings, lower levels of HDR-mediated gene modification were observed in HSCs and MPPs compared to progenitor cells. ZFN mRNA delivery and expression did not differ between HSC populations. HDR pathway genes were not differentially expressed in HSCs and progenitors at the time of electroporation (48 hrs post-stimulation). However, analysis of cytotoxic effects, as measured by flow cytometry and gene expression analysis of apoptotic pathways, revealed a higher sensitivity of HSCs to the electroporated ZFNs and oligonucleotide donor, resulting in -80% cell death compared to -30% observed in progenitors. Over-expression of the apoptosis regulator Bcl-2 ameliorated the treatmentassociated cytotoxicity in HSCs and resulted in a two- to three-fold more HSC posttreatment. The data indicate an elevated sensitivity to toxicity from the ZFN mRNA and oligonucleotide donor for HSCs compared to the more mature progenitor cells. Transient expression of Bcl-2 appears to preserve HSC survival after HDR-based gene editing, which increases the frequency of gene-corrected HSCs. These findings have implications for clinical development of HSC targeted gene therapy using genetically modified stem cells.

### Material & Methods

### Cells, culture and fluorescence activated cell sorting (FACS)

Adult human mobilized peripheral blood apheresis was purchased from the Division of Experimental Hematology and Cancer Biology, Cincinatti Children's Hospital Medical Center. CD34⁺ cells were isolated using the magnetic activated cell sorting (MACS) CD34⁺ enrichment kit (Miltenyi Biotec) and the CliniMACS system (Miltenyi Biotec), resulting in a purity of 96-98%. The cells were cultured for 2 days prior to electroporation in media containing X-vivo 15 (Lonza) supplemented with Penicillin/Streptomycin/L-glutamine and the cytokines recombinant human stem cell factor (SCF) (50ng/µl), recombinant human thrombopoietin (rhTPO) (50ng/µl) and recombinant human Flt3-ligand (rhFlt3-Ligand) (50ng/µl) (Peprotech). For FACS analysis, cells were immunostained with CD34-Pacific blue (or APC-Cy7), CD38-PeCy7, CD45RA-PerCP-Cy5.5 (or FITC), CD90-PE, DAPI (or 7AAD), Annexin V-FITC and Ki67-APC (Biolegend).

### Zinc Finger Nucleases

Developed by Sangamo Biosciences to target the region over the sickle mutation in exon 1 of beta-globin. A 101-bp single-stranded oligodeoxynucleotide was used as a homologous donor template, containing the corrective base (Eurofins MWG Operon).

### In vitro transcription of messenger RNA

Plasmids of ZFN and Bcl-2 were linearized with SpeI to serve as DNA templates and mRNA was synthesized using the mMessage mMachine T7 Ultra Kit (Ambion) and purified with RNeasy MinElute Cleanup Kit (Qiagen).

### Electroporation and gene modification

Electroporation of CD34⁺ cells was performed using the ECM 830 Electroporation System (Harvard Apparatus). 1 x 10⁶ cells were centrifuged and resuspended in 100µl of BTX solution together with the ZFN mRNA, donor and with or without (w/wo) BCL-2 mRNA. Genomic DNA was isolated using the PureLink Genomic DNA mini kit (Life Technologies). The beta globin locus was PCR-amplified with 3 sets of primers for targeted re-sequencing library preparation. The first set of primers (HBB F: 5'-atgcttagaaccgaggtagagttt- 3' (SEQ ID NO:1140) and HBB R: 5'- cctgagacttccacactgatg- 3' (SEQ ID NO:1141)) were designed to amplify beta globin outside of the donor region. The next set of primers were designed to have homology to the beta globin region, as well as P5 and P7 adapters for high throughput illumina sequencing, read 1 and read 2 sequence used for analysis, and a unique index to identify each sample. Illumina libraries were sequenced on an Illumina HiSeq 2500.

### Apoptosis gene expression

Cells were isolated for cellular RNA (RNeasy mini kit, Qiagen), converted to cDNA using PreAMP cDNA Synthesis Kit and primer mix (Qiagen) and analyzed for gene expression using RT² Profiler^{™} PCR Array Human Apoptosis 384HT (Qiagen, PAHS-3012ZE-2).

### NSG mice

NOD/SCID IL2R gamma^{-/-} (NSG) mice obtained from the Jackson Laboratory (Bar Harbor, ME, USA) were maintained by the UCLA Department of Laboratory Animal Medicine (DLAM) under protocols reviewed and approved by the UCLA Chancellor's Animal Research Committee (ARC# 2008-167). 1×10⁶ CD34⁺ cells were electroporated with ZFNs mRNA (5µg), Oligo (3µM), and with or without Bcl-2 mRNA (5µg).

### Results

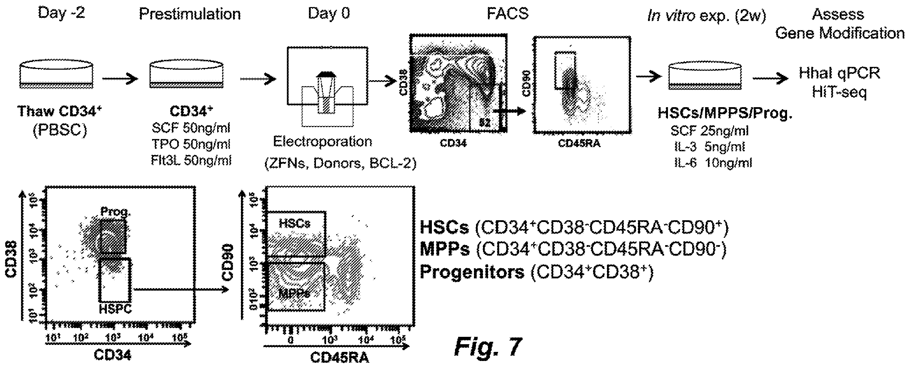

The data are depicted in Figures 7-13. Figure 7 provides a schematic depiction of the experimental design, and immunophenotype-defined populations.

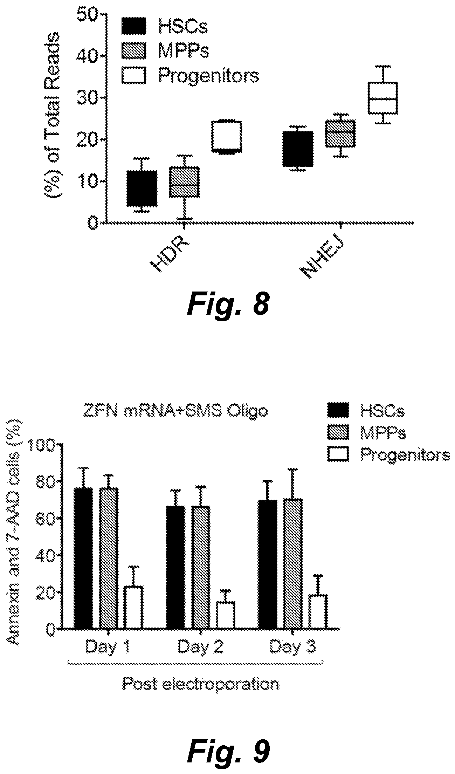

Figure 8 provides a HDR vs. NHEJ comparison in various cell populations. Peripheral blood (PB) CD34⁺ cells were pre-stimulated for two days and electroporated with ZFN mRNA and 3µM of an oligodeoxynucleotide homologous donor template. HDR and NHEJ analysis was determined by high throughput sequencing of the β-globin locus, n=6.

Figure 9 depicts cell death and apoptosis analysis of the different cell populations post-electroporation. To determine the cytotoxic effects of electroporation, ZFN mRNA and oligo donor in the three immunophenotypically identified populations, FACS analysis was performed to identify cells labeled with 7-AAD and AnnexinV, 20 hrs post-electroporation, n=5.

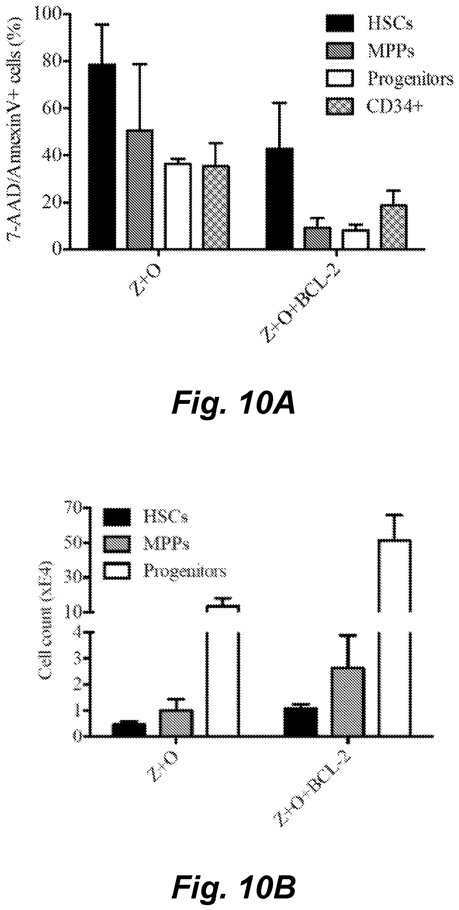

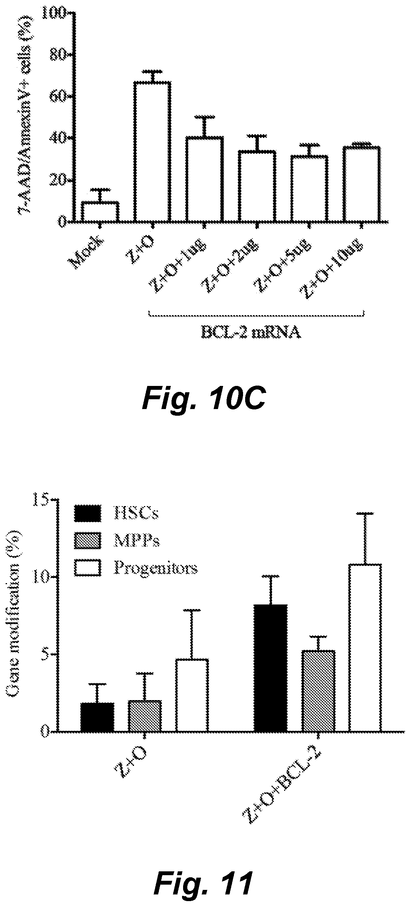

Figures 10A-10C depict the effect of transient overexpression of BCL-2 mRNA on cell toxicity as measured by flow cytometry and on the number of cells overall. As shown in FIG. 10A-10C, transient overexpression of BCL-2 mRNA decreases cell toxicity as measured by flow cytometry and increases the number of cells overall. The induced cytotoxicity was analyzed for hematopoietic stem cells (HSCs), multi-potent progenitors (MPPs) and progenitor cells 20hrs following electroporation of ZFN mRNA and oligonucleotide donor, with or without including BCL-2 mRNA. (a) Flow cytometry, identifying cells labeled with 7-aminoactinomycin D (7-AAD) and AnnexinV, n=4. (b) Cell count measured by FACS 20hrs post-electroporation, n=4. Starting cell conc.: 4E6 CD34⁺ cells (c) Cord blood CD34⁺ cells were tirated with 1, 2, 5 and 10µg of BCL-2 mRNA and the cell toxicity was analyzed 20hrs post-electroporation by flow cytometry identifying cells labeled with 7-AAD and AnnexinV, n=3.

Figure 11 depicts the effect of transient overexpression of BCL-2 mRNA on gene modification levels in HSC, MPP, and progenitor cells treated with ZFN mRNA and an oligonucleotide donor. The data in FIG. 11 show that transient overexpression of BCL-2 mRNA leads to higher gene modification levels in HSC, MPP and progenitor cells treated with ZFN mRNA and oligonucleotide donor. Site-specific HDR-mediated gene modification by quantitative reverse transcription-polymerase chain reaction (qRT-PCR) analysis of the *HhaI* restriction fragment length polymorphism (RFLP) (n=2) in the different cell populations, treated with or without BCL-2 mRNA, n=2.

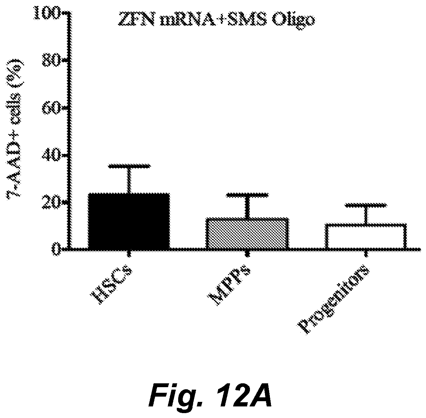

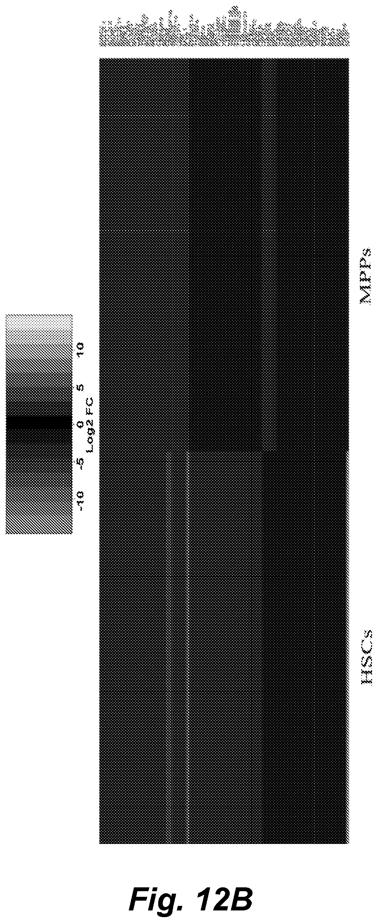

Figures 12A-12B depict apoptosis pathway gene expression analysis by quantitative polymerase chain reaction (qPCR) in human HSCs, MPPs and progenitor cells. To determine whether apoptosis pathway gene expression differences exist between the respective cell populations, 384 key genes involved in several apoptotic pathways were analyzed. CD34⁺ cells were pre-stimulated for two days, electroporated with ZFN mRNA (5µg) and oligonucleotide (oligo) donor (3µM) and thereafter sorted into HSCs, MPPs and progenitors cells by FACS, 6hrs post-electroporation, n=3. (a) Cell toxicity analyzed by FACS to identify cells labeled with 7-AAD, 6hrs post-electroporation. (b) HSCs, MPPs and progenitor cells were isolated to cellular RNA (6hrs post-electroporation), converted to cDNA and thereafter analyzed on the qRT-PCR, n=3. This initial study indicates a deficiency of the BCL-2 gene in HSCs and MPPs compared to progenitor cells, observed at this time point.

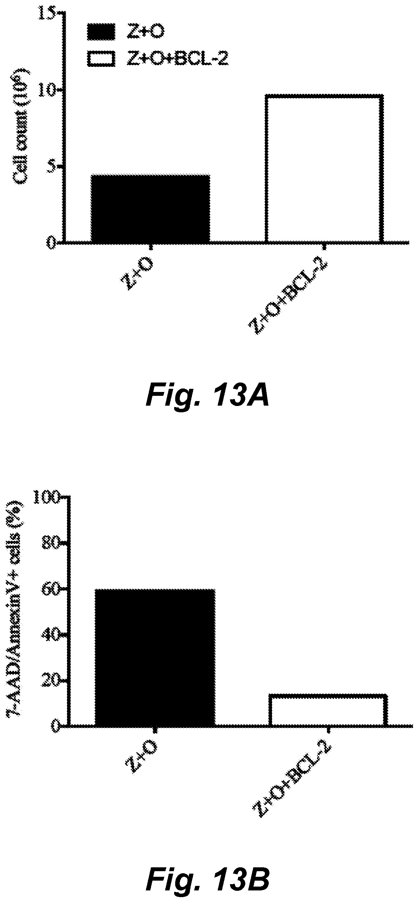

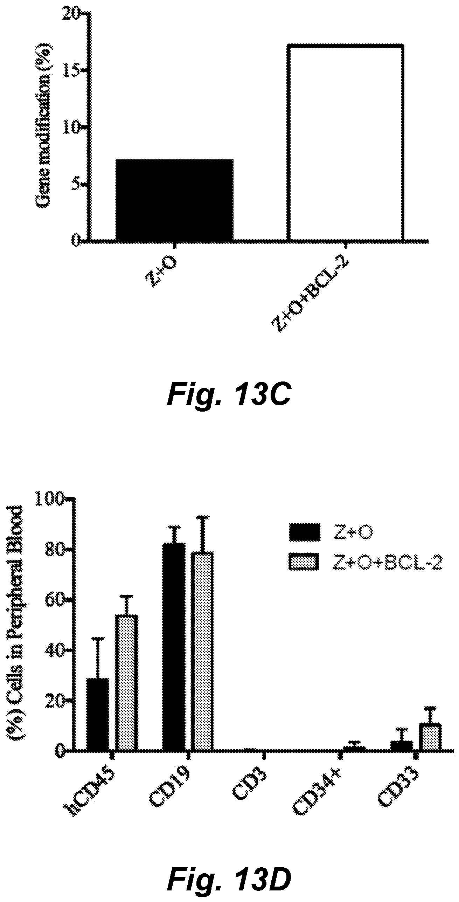

Figures 13A-D depict xenograft transplantations of female immune-deficient NSG mice. Two experimental arms with 10 million CD34⁺ cells respectively were pre-stimulated for two days and electroporated with ZFN mRNA (5µg) and oligo donor (3µM), w/wo BCL-2 mRNA (5µg). The following day, the cell count of the two treatment groups was measured by trypan blue (a) and cell death in the respective groups was measured (b).Gene modification was determined by qPCR, after 3 days of *in vitro* culture (c). Next, gene-modified CD34⁺ cells from each respective group were transplanted *in vivo* intravenously into NSG mice, where four mice received the remaining cells; Z+O group (1E6 viable gene modified cells/mouse) and the Z+O+BCL-2 group (2.25E6 viable gene modified cells/mouse). Two months after transplantation, the engraftment (%hCD45) and lineage analysis of human cells in peripheral blood was analyzed (d).

## Claims

1. A method of *ex vivo* editing of a target genomic DNA in a mammalian stem cell, the method comprising:
a) generating a modified mammalian stem cell that overexpresses the anti-apoptotic apoptosis regulator Bcl-2; and
b) contacting the modified mammalian stem cell with a genome editing composition comprising a genome editing endonuclease, or a nucleic acid encoding the genome editing endonuclease, wherein the genome editing endonuclease cleaves within a desired target sequence of the genomic DNA of the cell, wherein the genome-editing endonuclease enters the modified stem cell and edits the target genomic DNA;
wherein the mammalian cell is:
a pluripotent stem cell or an adult stem cell; and/or
a hematopoietic stem cell, a neural stem cell, a mesenchymal stem cell, or an induced pluripotent stem cell.

2. The method of claim 1, wherein the editing of the genomic DNA is an insertion of a sequence into the genomic DNA and/or a deletion of sequence from the genomic DNA, or where the modification of the genomic DNA is a substitution of one or more nucleotides of the target genomic DNA.

3. The method of claim 1 or claim 2, wherein the genome editing composition comprises:
a ribonucleoprotein (RNP) complex comprising a class 2 CRISPR/Cas endonuclease complexed with a corresponding CRISPR/Cas guide RNA that hybridizes to a target sequence within the genomic DNA of the cell;
a zinc finger nuclease; or
a TAL-effector DNA binding domain-nuclease fusion protein (TALEN).

4. The method of claim 3, wherein the genome editing composition comprises:
(i) a nucleic acid encoding a class 2 CRISPR/Cas endonuclease, and
(ii) a corresponding CRISPR/Cas guide RNA, or a nucleic acid encoding the corresponding CRISPR/Cas guide RNA, wherein the CRISPR/Cas guide RNA hybridizes to a target sequence within the genomic DNA of the cell.

5. The method of claim 3, wherein the class 2 CRISPR/Cas endonuclease cleaves the genomic DNA, resulting in editing of the target genomic DNA.

6. The method according to any one of claims 3-5, wherein:
the class 2 CRISPR /Cas endonuclease is a type II CRISPR/Cas endonuclease; and/or
the class 2 CRISPR /Cas endonuclease is a Cas9 polypeptide and the corresponding CRISPR/Cas guide RNA is a Cas9 guide RNA; and/or
the class 2 CRISPR /Cas endonuclease is a type V or type VI CRISPR/Cas endonuclease; and/or
the class 2 CRISPR/Cas polypeptide is a Cpf1 polypeptide, a C2c1 polypeptide, a C2c3 polypeptide, or a C2c2 polypeptide.

7. The method according to any one of claims 3-6, wherein the genome editing composition comprises a donor template nucleic acid.

8. The method according to any one of claims 1-7, wherein the Bcl-2 polypeptide comprises an amino acid sequence having at least 85% amino acid sequence identity to the amino acid sequence set forth in FIG. 1A or FIG. 1B.

9. The method of claim 8, wherein the Bcl-2 polypeptide comprises an amino acid sequence having the sequence set forth in FIG. 1A or FIG 1B.

10. The method according to any one of claims 1-9, wherein:
the apoptosis regulator is transiently overexpressed; or
the apoptosis regulator is overexpressed for a period of time ranging from about 1 hour to about 48 hours; or
the apoptosis regulator is overexpressed for a period of time ranging from about 48 hours to about 72 hours.

11. The method according to any one of claims 1-10, wherein the apoptosis regulator is overexpressed by at least 50% over background.

12. The method of claim 5, wherein the Cas9 guide RNA is a single guide RNA (sgRNA).

## Patentansprüche

1. Verfahren zur Ex-vivo-Bearbeitung einer Zielgenom-DNA in einer Säugetierstammzelle, wobei das Verfahren Folgendes umfasst:
a) das Erzeugen einer modifizierten Säugetierstammzelle, die den anti-apoptotischen Apoptose-Regulator Bcl-2 überexprimiert; und
b) das Inkontaktbringen der modifizierten Säugetierstammzelle mit einer Genombearbeitungszusammensetzung, die eine Genombearbeitungsendonuclease oder eine Nucleinsäure, die für die Genombearbeitungsendonuclease kodiert, umfasst, wobei die Genombearbeitungsendonuclease in einer gewünschten Zielsequenz der Genom-DNA der Zelle spaltet, wobei die Genombearbeitungsendonuclease in die modifizierte Stammzelle eindringt und die Zielgenom-DNA bearbeitet;
wobei die Säugetierstammzelle Folgendes ist:
eine pluripotente Stammzelle oder eine adulte Stammzelle; und/oder
eine hämatopoetische Stammzelle, eine neurale Stammzelle, eine mesenchymale Stammzelle oder eine induzierte pluripotente Stammzelle.

2. Verfahren nach Anspruch 1, wobei das Bearbeiten der Genom-DNA eine Insertion einer Sequenz in die Genom-DNA und/oder eine Deletion einer Sequenz von der Genom-DNA ist oder wobei die Modifikation der Genom-DNA eine Substitution von einer oder mehreren Nucleotiden der Zielgenom-DNA ist.

3. Verfahren nach Anspruch 1 oder Anspruch 2, wobei die Genombearbeitungszusammensetzung Folgendes umfasst:
einen Ribonucleoprotein- (RNP-) Komplex, der eine Klasse-2-CRISPR/Cas-Endonuclease im Komplex mit einer entsprechenden CRISPR/Cas-Guide-RNA umfasst, die an eine Zielsequenz in der Genom-DNA der Zelle hybridisiert;
eine Zinkfingernuclease; oder
ein TAL-Effektor-DNA-Bindungsdomäne-Nuclease-Fusionsprotein (TALEN).

4. Verfahren nach Anspruch 3, wobei die Genombearbeitungszusammensetzung Folgendes umfasst:
(i) eine Nucleinsäure, die für eine Klasse-2-CRISPR/Cas-Endonuclease kodiert, und
(ii) eine entsprechende CRISPR/Cas-Guide-RNA oder eine Nucleinsäure, die für die entsprechende CRISPR/Cas-Guide-RNA kodiert, wobei die CRISPR/Cas-Guide-RNA an eine Zielsequenz in der Genom-DNA der Zelle hybridisiert.

5. Verfahren nach Anspruch 3, wobei die Klasse-2-CRISPR/Cas-Endonuclease die Genom-DNA spaltet, was zu einer Bearbeitung der Zielgenom-DNA führt.

6. Verfahren nach einem der Ansprüche 3 bis 5, wobei:
die Klasse-2-CRISPR/Cas-Endonuclease eine Typ-II-CRISPR/Cas-Endonuclease ist; und/oder
die Klasse-2-CRISPR/Cas-Endonuclease ein Cas9-Polypeptid und die entsprechende CRISPR/Cas-Guide-RNA eine Cas9-Guide-RNA ist; und/oder
die Klasse-2-CRISPR/Cas-Endonuclease eine Typ-V- oder Typ-VI-CRISPR/Cas-Endonuclease ist; und/oder
das Klasse-2-CRISPR/Cas-Polypeptid ein Cpf1-Polypeptid, ein C2c1-Polypeptid, ein C2c3-Polypeptid oder ein C2c2-Polypeptid ist.

7. Verfahren nach einem der Ansprüche 3 bis 6, wobei die Genombearbeitungszusammensetzung eine Donormatrizen-Nucleinsäure umfasst.

8. Verfahren nach einem der Ansprüche 1 bis 7, wobei das Bcl-2-Polypeptid eine Aminosäuresequenz mit zumindest 85 % Aminosäuresequenzidentität mit der in FIG. 1A oder FIG. 1B dargelegten Aminosäuresequenz umfasst.

9. Verfahren nach Anspruch 8, wobei das Bcl-2-Polypeptid eine Aminosäuresequenz mit der in FIG. 1A oder FIG. 1B dargelegten Sequenz umfasst.

10. Verfahren nach einem der Ansprüche 1 bis 9, wobei:
der Apoptose-Regulator vorübergehend überexprimiert wird; oder
der Apoptose-Regulator über einen Zeitraum im Bereich von etwa 1 h bis etwa 48 h überexprimiert wird; oder
der Apoptose-Regulator über einen Zeitraum im Bereich von etwa 48 h bis etwa 72 h überexprimiert wird.

11. Verfahren nach einem der Ansprüche 1 bis 10, wobei der Apoptose-Regulator um zumindest 50 % über Hintergrund überexprimiert wird.

12. Verfahren nach Anspruch 5, wobei die Cas9-Guide-RNA eine Einzel-Guide-RNA (sgRNA) ist.

## Revendications

1. Procédé d'édition ex *vivo* d'un ADN génomique cible dans une cellule souche de mammifère, le procédé comprenant les étapes consistant à :
a) générer une cellule souche de mammifère modifiée qui surexprime le régulateur d'apoptose anti-apoptotique Bcl-2 ; et
b) mettre en contact la cellule souche de mammifère modifiée avec une composition d'édition de génome comprenant une endonucléase d'édition de génome, ou un acide nucléique codant pour l'endonucléase d'édition de génome, dans lequel l'endonucléase d'édition de génome se clive dans une séquence cible souhaitée de l'ADN génomique de la cellule, dans lequel l'endonucléase d'édition de génome pénètre dans la cellule souche modifiée et édite l'ADN génomique cible ;
dans lequel la cellule de mammifère est :
une cellule souche pluripotente ou une cellule souche adulte ; et/ou
une cellule souche hématopoïétique, une cellule souche neurale, une cellule souche mésenchymateuse ou une cellule souche pluripotente induite.

2. Procédé selon la revendication 1, dans lequel l'édition de l'ADN génomique est une insertion d'une séquence dans l'ADN génomique et/ou une délétion de séquence depuis l'ADN génomique, ou dans lequel la modification de l'ADN génomique est une substitution d'un ou plusieurs nucléotides de l'ADN génomique cible.

3. Procédé selon la revendication 1 ou la revendication 2, dans lequel la composition d'édition de génome comprend :
un complexe de ribonucléoprotéine (RNP) comprenant une endonucléase CRISPR/Cas de classe 2 complexée avec un ARN guide CRISPR/Cas correspondant qui s'hybride à une séquence cible à l'intérieur de l'ADN génomique de la cellule ;
une nucléase à doigt de zinc ; ou
une protéine de fusion nucléase d'effecteur TAL et de domaine de liaison à l'ADN-domaine de liaison a l'ADN (TALEN).

4. Procédé selon la revendication 3, dans lequel la composition d'édition de génome comprend :
(i) un acide nucléique codant pour une endonucléase CRISPR/Cas de classe 2, et
(ii) un ARN guide CRISPR/Cas correspondant, ou un acide nucléique codant pour l'ARN guide CRISPR/Cas correspondant, l'ARN guide CRISPR/Cas s'hybridant à une séquence cible dans l'ADN génomique de la cellule.

5. Procédé selon la revendication 3, dans lequel l'endonucléase CRISPR/Cas de classe 2 clive l'ADN génomique, en ayant pour résultat l'édition de l'ADN génomique cible.

6. Procédé selon l'une quelconque des revendications 3 à 5, dans lequel :
l'endonucléase CRISPR/Cas de classe 2 est une endonucléase CRISPR/Cas de type II ; et/ou
l'endonucléase CRISPR/Cas de classe 2 est un polypeptide Cas9 et l'ARN guide CRISPR/Cas correspondant est un ARN guide Cas9 ; et/ou
l'endonucléase CRISPR/Cas de classe 2 est une endonucléase CRISPR/Cas de type V ou de type VI ; et/ou
le polypeptide CRISPR/Cas de classe 2 est un polypeptide Cpf1, un polypeptide C2c1, un polypeptide C2c3 ou un polypeptide C2c2.

7. Procédé selon l'une quelconque des revendications 3 à 6, dans lequel la composition d'édition de génome comprend un acide nucléique de matrice donneur.

8. Procédé selon l'une quelconque des revendications 1 à 7, dans lequel le polypeptide Bcl-2 comprend une séquence d'acides aminés présentant au moins 85 % d'identité de séquence d'acides aminés avec la séquence d'acides aminés présentée dans la figure 1A ou la figure 1B.

9. Procédé selon la revendication 8, dans lequel le polypeptide Bcl-2 comprend une séquence d'acides aminés présentant la séquence décrite dans la figure 1A ou la figure 1B.

10. Procédé selon l'une quelconque des revendications 1 à 9, dans lequel :
le régulateur d'apoptose est surexprimé de manière transitoire ; ou
le régulateur d'apoptose est surexprimé pendant une période de temps dans la plage d'environ 1 heure à environ 48 heures ; ou
le régulateur d'apoptose est surexprimé pendant une période de temps dans la plage d'environ 48 heures à environ 72 heures.

11. Procédé selon l'une quelconque des revendications 1 à 10, dans lequel le régulateur d'apoptose est surexprimé d'au moins 50 % sur le fond.

12. Procédé selon la revendication 5, dans lequel l'ARN guide Cas9 est un ARN guide unique (ARNsg).
